# EUROPEAN PATENT SPECIFICATION

(11) **EP 1 811 914 B1**
(45) Date of publication and mention of the grant of the patent: **01.07.2015**
(21) Application number: 05798758.8
(22) Date of filing: 21.09.2005
(51) Int. Cl.: A61B 19/00, A61F 2/12

(54) **TISSUE EXPANSION DEVICES**
GEWEBEEXPANSIONSVORRICHTUNGEN
DISPOSITIFS D'EXPANSION TISSULAIRE

(30) Priority: 21.09.2004 US 612018 P; 09.06.2005 US 688964 P
(43) Date of publication of application: 01.08.2007
(73) Proprietor: Shalon Ventures Inc., Palo Alto, CA 94301 (US)
(72) Inventor: SHALON, Tadmor, Palo Alto, CA 94301 (US); JACOB, Daniel, Mountain View, CA 94040-3832 (US); SAWAN, Samuel, P., Tyngsboro, MA 01879 (US); CAHILL, Sean, S., Palo Alto, CA 94306 (US); SOKOLOFF, Norman, Los Altos Hills, CA 94024 (US); WETENKAMP, Scott, los Altos, CA 94022 (US)
(74) Representative: Dennemeyer & Associates S.A.
(86) International application number: PCT/US2005/033664
(87) International publication number: WO 2006/034273

(56) References cited:
- WO-A-01/47441
- WO-A-95/03752
- WO-A-97/27829
- WO-A-98/50100
- WO-A-2004/066812
- WO-A1-80/00302
- FR-A- 2 615 397
- US-A- 4 550 720
- US-A- 5 005 591
- US-A- 5 376 117
- US-A1- 2003 074 084
- US-B1- 6 875 233

## Description

### FIELD OF THE INVENTION

The present invention relates to implantable tissue expansion devices.

### BACKGROUND OF THE INVENTION

A deficit of normal tissue in a subject may result from, for example, bums, tumor resection surgery (e.g. mastectomy), or congenital deformities. Often, the tissue in deficit is skin and/or underlying connective tissue. The tissue in deficit can also be an intrabody duct (e.g. urethras or GI tract).

One method of correcting skin deficit is to stimulate creation of new skin. Implantation of a device that expands and stretches the existing skin causes a growth response in which new skin is created. While the exact physiologic mechanism of this response remains unclear, clinical success has been reported over many years.

The first report of tissue expansion was in 1956 by Charles Neumann (Plastic & Reconstructive Surgery; Vol 19 (2); 124-130) who implanted a rubber balloon attached to a percutaneous tube to enable intermittent expansion for the purpose of reconstructing a partially amputated ear. Since that time, the idea of tissue expansion devices has undergone commercial development.

Most commercially available tissue expanders function as an implantable balloon with an extracorporeal or imbedded valve that allows periodic inflation. Typically, it is a doctor that performs the inflation. Since the inflation events are relatively infrequent, a significant inflation pressure is typically applied at each doctor's visit in order to achieve maximum effect from each visit. As a result of this inflation pressure during a clinic visit, a relatively sudden tissue stretch occurs. This may cause subjects to suffer discomfort and/or tissue ischemia. The relatively large inflation pressure can also adversely affect underlying structures (e.g. cause concavities in underlying bone). In addition, high pressure may create restrictive capsules around the implant and/or cause tissue failure. Some previously available alternatives used a needle for inflation or filling, creating a potential source of infection.

In order to overcome such issues, continuously expanding devices have been developed. For example, osmotic expanders have been reported by Austad in 1979, Berge in 1999, and Olbrisch in 2003 (see US Patent Nos. 5,005,591 and 5, 496368). A commercial version is available from Osmed Corp. in a limited range of sizes. These devices use a polymeric osmotic driver to expand a silicone implant by absorbing interstitial fluid (ISF). A potential problem of such devices is the lack of control or adjustability after implantation with respect to expansion variable such as pressure, volume, onset of expansion, and end of expansion once they have been deployed.

US Patent No. 6,668,836 to Greenberg et al describes a method for pulsatile expansion of tissue using an external hydraulic pump. The external hydraulic pump is bulky and may lead to negative subject reactions. The percutaneous attachment reduces subject mobility and may be a source of contamination.

US Patent No. 4,955,905 to Reed teaches an external monitor for pressure of an implanted fluid filled tissue expansion device.

US Patent Nos. 5,092,348 and 5,525,275 to Dubrul and Iverson respectively teach implantable devices with textured surfaces.

US Patent Application No. 2004/0,147,953 by Gebedou teaches a device which relies upon an internal mechanical force as a means of avoiding use of fluids for tissue expansion.

US patients 6,264,936; 6,180,584; 6,126,931; 6,030,632; 5,869,073; 6,849,311 and 5,817,325 deal generally with the concept of antimicrobial coatings.

In WO 80/00302 A1, there is described a tissue expansion device according to the preamble of claim 1 comprising: an expandable compartment adapted for implanting in a body of a subject, and a compressed gas source operably communicating with the expandable compartment for inflation thereof by transfer of a gas thereto.

A tissue expansion device, as described in the preamble of claim 1, is disclosed in US 6 875 233.

It is against the background, and the limitations and problems associated therewith, that the present invention has been developed.

To achieve this, the tissue expansion device of the invention is characterized by the features claimed in the characterizing part of claim 1.

According to the present invention, the compressed gas source is positioned completely within the expandable compartment.

Advantageous embodiments of the invention are claimed in the subclaims.

### SUMMARY OF THE INVENTION

An aspect of some embodiments of the present invention relates to a self contained implantable tissue expansion device including an expandable compartment and fill source, optionally, the fill source is a gas source. Optionally, the expandable compartment is inflated by gas from the gas source.

In an exemplary embodiment of the invention, the tissue expansion device is provided as an expanding breast implant. Optionally, the breast implant stretches skin and/or sub-dermal tissue of a damaged breast (e.g. post mastectomy) to more closely conform to a contra-lateral breast which is not damaged. Optionally, the tissue expansion implant is provided as a temporary measure and is replaced by a permanent implant once a desired degree of tissue expansion is achieved. Optionally, the tissue expansion implant serves as a long term cosmetic implant. In an exemplary embodiment of the invention, the breast tissue expansion, implant in converted to a long term cosmetic implant.

In an exemplary embodiment of the Invention, the tissue expansion device is optionally employed to grow new skin and/or underlying tissue to permit repair at another location. In an exemplary embodiment of the invention, the new skin and/or underlying tissue is harvested and transferred to a new location as an autologous graft

Optionally, the expandable compartment may be constructed of an elastic balloon and/or an inelastic deformable shell. In an exemplary embodiment of the invention, use of elastic materials in combination with inelastic deformable materials allows the device to conform to a natural body contour during expansion. Optionally, modeling of the compartment to a specific subject permits the device to conform to a body contour of that subject. Modeling may be, for example, to a contra-lateral body part (e.g. breast) or to a body part prior to surgery.

In an exemplary embodiment of the invention, a transfer of gas into the expandable compartment is regulated. Optionally, regulation may be via a valve and/or actuator. Optionally, transfer may be regulated by sequential and/or concurrent release of gas from one or more of a plurality of containers, each container containing a fixed amount of gas.

Optionally, the gas source and/or valve and/or actuator are contained within the expandable compartment. Optionally, this configuration protects these components and/or adjacent tissues. Optionally, this configuration prevents these components from disrupting a natural contour of the body of the subject.

A particular feature of some embodiments of the invention is that change in volume of the expander can be made gradual. Optionally, gradualness is used to prevent discomfort and/or ischemia and/or other adverse effects of tissue expansion. Optionally, a small size or shape of rigid components of the device reduces disruption of body contours and/or provides a more natural feeling. Optionally, a natural body contour increases comfort of the subject. In an exemplary embodiment of the invention, graduality is provided by relatively slow ingress of fluid into the expandable compartment Optionally, slow ingress is provided by relatively slow flow rates and/or by providing multiple small incremental additions of fluid to the compartment In an exemplary embodiment of the invention, over a two week period at least 5, 20, 50, 100, 1000 or intermediate or greater numbers of incremental additions are performed. Alternatively or additionally, a slow flow rate having a maximum of 20 ml/s, optionally 10 ml/s, optionally 5 ml/s, optionally 2.5 ml/s, optionally 1 ml/s, optionally 0.5 ml/s, optionally 0.1 ml/s, optionally 0.001 ml/s, optionally 0.0001 ml/s or intermediate or smaller values. Optionally, the use of a low flow rate provides safely in that sudden rupture is less likely to occur without warning. In on exemplary embodiment of the invention, graduality is provided in that an actual change in volume of the compartment is gradual. In an exemplary embodiment of the invention, the use of gas at low pleasures relative to the mechanical characteristics of surrounding tissue (e.g. expansion rate and/or elastic limit and/or breaking limit) allows the compartment to expand in a manner commensurate with an ability of the surrounding tissue to favorably respond to such expansion. In some cases this means that the volume of the compartment changes less than a volume of an added increment of gas while the internal compartment pressure increases slightly. For example compartment pressure may increase by 10%, optionally 7.5%, optionally 5%, optionally 2.5%, optionally 1%, optionally 0.5% or less and slowly return to a pre-inflation event pressure as a tissue expands to accommodate the newly introduced gas.

In an exemplary embodiment of the invention, the device is powered by a power source. Optionally, the power source drives an actuator for flow regulation. Optionally, the power source is regulatable. Optionally, regulation of the power source provides a means for control a fill rate of the expandable compartment. Optionally, regulation of the power source increases a safety level of the device. In an exemplary embodiment of the invention, the power source is physically separate from the device and must be brought close to the device in order to cause a transfer of gas from the gas source to the expandable compartment Optionally, the power source is provided in a separate extracorporeal control device. Optionally, an external magnet functions as the power source and bringing the magnet into close proximity to a ferromagnetic or magnetic valve cause a flow of gas through a valve.

In an exemplary embodiment of the invention, expansion of the expandable compartment is via an open-loop expansion mechanism. Optionally, gas is continuously released into the expandable compartment In an exemplary embodiment of the invention, expansion of the expandable compartment is performed according to a program in which gas is periodically transferred to the expandable compartment. In an exemplary embodiment of the invention, a subject in whom the device is implanted at least partially controls expansion of the expandable compartment. Optionally, this partial control includes control of timing and/or control of magnitude of gas transfer. In an exemplary embodiment of the invention, the actuator is subject to a closed regulatory loop based on sensor data and/or human input. Optionally, no conscious cooperation of the subject is required. Optionally, the transfer of gas into the expandable compartment is gradual enough that a subject in whom the device is implanted does not perceive the expansion as it occurs. In an exemplary embodiment of the invention, implementation of a controlled release of gas reduces the need for doctor's visits.

In an exemplary embodiment of the invention, sensors are provided in the device to measure parametric data. Optionally, data pertains to the expandable compartment and/or a subject response and/or a valve parameter and/or an actuator parameter. In an exemplary embodiment of the invention, patient response is ascertained by a measure of blood perfusion of tissue covering the implantable tissue expansion device. Optionally, the device includes a data processing unit (e.g. digital microprocessor and/or analog circuitry and/or a mechanical circuit) to provide an interface between a data sensor and an actuator and/or to store and/or transmit data acquired by sensors. In an exemplary embodiment of the invention, the data sensors are used to control expansion of the compartment by imposing a feedback loop on the actuator. Optionally, stored data is analyzed with respect to a single subject and/or as part of a multi-subject database.

In an exemplary embodiment of the invention, the actuator is responsive to a signal originating outside the body from a separate control unit. Optionally, the signal is delivered to the actuator and/or the microprocessor without a physical percutaneous link. Optionally, the control unit must be held close to the tissue expansion device during operation.

In an exemplary embodiment of the invention, a power source for the tissue expansion device at least partially resides in the control unit. Optionally, this facilitates reduction of a size and/or weight of the implantable tissue expansion device. Optionally, power is transferred to the device from the controller through matching RF coils. Optionally, the RF coils are designed with a short signal range (e.g. 25 mm or less) and specific frequency (e.g., 11 MHz) to reduce the likelihood of accidental inflation of the expandable compartment. Optionally, the control unit is small and portable and may be operated by either a doctor or by the subject in whom the device is implanted.

An aspect of some embodiments of the present invention relates to a wireless controller configured to provide a reliable means of controlling a transfer of gas from a gas source to an expandable compartment of an implantable tissue expansion device. Optionally, a signal source in the controller may be keyed to one or more devices in order to control who controls which device(s). In an exemplary embodiment of the invention, the wireless controller is operable by a subject in whom the implantable tissue expansion device is implanted. In an exemplary embodiment of the invention, a doctor uses a single wireless controller to operate devices implanted in several subjects. Optionally, the wireless controller additionally includes a signal receiver which may receive data. Optionally the data pertains to device function and/or subject response. In an exemplary embodiment of the invention, a doctor uses a single control device to control tissue expansion and/or collect data from multiple subjects. Optionally, data collection is automated.

In an exemplary embodiment of the invention, a subject is issued a wireless controller. Optionally, the subject assumes at least partial responsibility for management of a transfer of gas from a gas source to an expandable compartment of an implantable tissue expansion device implanted in their body. Optionally, the wireless controller relays gathered information on device performance and/or patient response to a remote location for medical supervision and/or statistical analysis. The subject may initiate a transfer of gas to the expandable compartment according to a schedule and/or until a discomfort threshold is reached and/or according to their convenience. Optionally, subject accessible feedback is provided to encourage active participation.

In an exemplary embodiment of the invention, a subject with an implanted tissue expander periodically uploads data from their device to a remote server. Optionally, the remote server issues an instruction to the device based upon analysis of the uploaded data. Optionally, data upload occurs via a telephone connection. Optionally medical personnel review uploaded data. Optionally, a treatment plan may be modified without a clinic visit.

An aspect of some embodiments of the present invention relates to a flow rate restrictor, optionally suitable for use at low flow rates. The restrictor includes a narrow orifice (e.g. capillary tube or tortuous path membrane) which limits the rate at which gas may flow through the restrictor. Optionally, an actuator further restricts flow by opening and closing the narrow orifice.

In an exemplary embodiment of the invention, a silicon narrow orifice restrictor with an elastomeric sealing surface is employed in conjunction with an actuator. Optionally, the force applied through the sealing surface to prevent a flow of gas through the narrow orifice restrictor is related to an orifice diameter and/or distance (e.g. capillary tube lengthy or tortuous path length) and/or a pressure in the gas source. In an exemplary embodiment of the invention, this type of arrangement permits a small applied force (e.g. less than 10 grams) to stop flow caused by hundreds of PSI of gas pressure in the gas source.

In an exemplary embodiment of the invention, a regulatable valve is supplied by subjecting the actuator to stringent control, optionally variable control. Optionally, a small controlled translational motion induced by a small power input is sufficient to switch between no flow and flow.

In an exemplary embodiment of the invention, an antimicrobial coating is applied to at least a portion of the tissue expansion device to prevent contamination and/or infection. Optionally, the coating is a non-eluting coating (e.g. Surfacine from SDC, Tyngsboro MA or related compounds), an eluting coating (e.g. silver or an antibiotic) or a combination thereof.

In an exemplary embodiment of the invention, external surfaces of the device are treated with an antibacterial substance. Optionally, treatment is with a non-eluting coating and/or an eluting coating. In an exemplary embodiment of the invention, application of an antimicrobial coating prevents or retards formation of a biofilm. Alternatively or additionally, an antimicrobial coating prevents a coated portion of the device introducing an infection into the body.

According to an aspect of some embodiments of the invention an implantable device is anchored to prevent shifting after implantation. Optionally, the device may be a tissue expansion device and/or a long term cosmetic implant. Shifting may alter a subject's appearance in an undesirable fashion and/or change a tissue expansion site to an undesired location. Optionally, anchoring is to a body tissue. Optionally, an anterior studded surface is employed for anchoring. Optionally, projecting studs penetrate the overlying pectoralis musola. Optionally, this projection prevents movement of the device with respect to the muscle. Optionally, studs of 2-3 mm in length are employed. In an exemplary embodiment of the invention, 6-10 studs are sufficient for stabilization. Optionally, the studs are resorbable. In an exemplary embodiment of the invention, once a capsule has formed to stabilize the position of the device, the studs are resorbed. In an exemplary embodiment of the invention, anchoring stabilizes a position of a device implanted In a breast oven if release of the medial portion of the pectoralis major origin is partially preserved.

According to an aspect of some embodiments of the invention, there is provided a database which correlates subject response to objective operational data on an implanted tissue expansion device 300. Optionally, subject response may be objective and/or subjective.

In an exemplary embodiment of the invention, there is provided a tissue expansion comprising:
(a) an expandable compartment adapted for implanting in a body of a subject; and
(b) a gas source adapted for implanting in a body of a subject and operably connected to said expandable compartment for inflation thereof by transfer of a gas thereto.

Optionally, the expandable compartment is at least partially constructed from an elastic material.

Optionally, the expandable compartment is at least partially constructed from an inelastic material.

Optionally, the expandable compartment is at constructed completely from an inelastic material

Optionally, the gas source is contained within said expendable compartment

Optionally, the expandable compartment is filled from said gas source via an open loop expansion mechanism.

Optionally, the open loop expansion lasts 7 to 180 days.

Optionally, the gas source contains a pressurized gas.

Optionally, the device includes a regulator to regulate a flow of said gas from said gas source into said expandable compartment

Optionally, the regulator includes a flow restriction pathway.

Optionally, the regulator includes a flow restriction membrane to restrict a flow of gas from said gas source to said expandable compartment.

Optionally, the regulator includes an actuator, said actuator designed and constructed to alternately permit and deny a flow of gas through said regulator.

Optionally, the regulator includes a valve to restrict a flow of gas from said gas source to said expandable compartment.

Optionally, the regulator includes at least one data processing circuit.

Optionally, the data processing circuit is an analog data processing module.

Optionally, the data processing circuit is a digital circuit.

Optionally, the regulator is subject to mechanical feedback.

Optionally, the regulator is responsive to an input signal so that said regulator implements a feedback loop.

Optionally, the input signal includes an operational command originating from an external controller.

Optionally, the regulator response persists only as long as said operational command.

Optionally, the regulator response persists after said operational command ceases.

Optionally, the external controller issues a compliance reminder, said compliance reminder indicating that said operational command should be delivered..

Optionally, the input signal includes an output signal from at least one sensor.

Optionally, the device includes: a parametric sensor configured to measure at least one parameter of the subject.

Optionally, the at least one parameter of the subject includes a measure of a degree of tissue perfusion.

Optionally, the device includes a parametric sensor configured to measure at least one parameter of the expandable compartment.

Optionally, the at least one parameter of the expandable compartment includes a measure of a gas pressure within said compartment.

Optionally, the gas source comprises a plurality of gas sources.

Optionally, each source in said plurality of gas sources is configured for selectable discharge according to an open loop.

Optionally, not all sources in said plurality of sources contain an identical amount of gas.

Optionally, the device includes: (c) a power source configured to provide a power output to facilitate said transfer

Optionally, the power source includes a battery.

Optionally, the battery resides within said expandable compartment.

Optionally, the power source resides outside the device and transmits power in a wireless manner to the device.

Optionally, the device includes: (c) a power source configured to provide a power output for at least one of data collection, transmission and storage.

Optionally, the power source resides outside the device and transmits power in a wireless manner to the device.

Optionally, the power source supplies power for transfer of data from the device to a location outside the device.

Optionally, the device includes: (c) an outer shall surrounding said expandable compartment.

Optionally, the expandable compartment comprises an elastic balloon.

Optionally, the expandable compartment comprises an inelastic deformable container.

Optionally, the outer shell comprises an elastic balloon.

Optionally, the outer shell comprises an inelastic doformable container.

Optionally, the inelastic deformable container expands to conform to a natural contour of a body part.

Optionally, the device includes: (c) a release valve to release gas from said expandable compartment.

Optionally, the release of gas includes release into a body tissue.

Optionally, the release mechanism operates at a set point between 150 and 300 mm of mercury.

Optionally, the device includes a studded surface comprising a plurality of studs to inhibit relative translational motion between said studded surface and an adjacent tissue layer.

Optionally, the studded surface is an anterior surface of a breast expander and said adjacent tissue layer includes at least a portion of a pectoralis muscle.

Optionally, the studs protrude from said surface between 0.5 and 5 mm.

Optionally, the plurality of studs includes 2 to 500 studs.

Optionally, the device is designed and configured as a breast expansion device.

Optionally, the device is designed and configured to discharge gas from said gas source into said expandable compartment during a period of at least 7 days.

Optionally, the device is designed and configured to provide an expansion pressure of 10 to 200 mm of mercury to an adjacent tissue.

Optionally, the device additionally includes an antimicrobial coating.

Optionally, said antimicrobial coating coats at least a portion of an inner surface of said expandable compartment.

Optionally, said antimicrobial coating coats at least a portion of an outer surface of said expandable compartment

Optionally, said expandable compartment comprises at least two expandable compartments.

Optionally, the device has a pre-implantation shelf life of at least 30 days.

In an exemplary embodiment of the invention, there is provided a regulator to regulate a flow of gas from a pressurized gas source into a low pressure area, the mechanism comprising:
(a) a narrow outlet adapted for connection to a gas source with a pressure of at least 20 PSI;
(b) a seal applicable to said orifice to stop the flow of gas from the high pressure gas source into a low pressure area;
(c) an actuatable component capable of selectively applying and removing a force to said seal thereby selectively preventing and allowing gas flow.

Optionally, a total volume of said mechanism is in the range of 4 mm³ to 20 cm³.

Optionally, said force is less than 10 gram-force.

Optionally, a total volume of said mechanism is in the range of 4 mm³ to 20 cm³.

Optionally, the force is less than 10 gram-force.

Optionally, the actuatable component requires a power input of less than 500 mW.

Optionally, the gas source contains a pressure in the range of 150 two 1500 PSI.

Optionally, the outlet has a cross-sectional are less than 0.05 mm².

Optionally, the mechanism additionally includes:
(d) an elongate narrow path to said narrow orifice, said elongate path to restrict said gas flow rate by friction.

Optionally, the force applied by said actuatable piston causes deformation of said elastomeric seal to seal said narrow orifice.

Optionally, the mechanism includes a controller to control said actuatable piston.

Optionally, the controller includes circuitry.

Optionally, the controller includes a mechanical control device.

Optionally, the mechanism includes an on/off control.

Optionally, the actuatable component at least partially relies upon an electric current for actuation

Optionally, the actualable component at least partially relies upon a magnetic field for actuation

Optionally, the actuatable component at least partially relies upon a spring for actuation

Optionally, the actuatable component at least partially relies upon a heat deformable element for actuation.

In an exemplary embodiment of the invention, there is provided a regulation mechanism to regulated a flow of gas from a pressurized gas source into a low pressure area, the mechanism comprising
(a) a membrane which restricts a gas flow rate from the gas source with a pressure of at least 20 PSI; and
(b) a valve with a pressure set point, said pressure set point lower than a pressure in the gas source and higher than a pressure in the low pressure area.

Optionally, the mechanism includes:
(c) a switch to turn the flow of gas an and off.

In an exemplary embodiment of the invention, there is provided a tissue expansion device comprising:
(a) an expandable compartment adapted for implanting in a body of a subject; and
(b) a fill source adapted for implanting in a body of a subject and operably connected to said expandable compartiment for inflation thereof by transfer of a gas thereto.

Optionally, said fill source is a regulatable fill source designed and constructed to fill said fill source at a controlled rate.

Optionally, the device includes comprising a controller, said controller exercising control over said fill mechanism to control a fill rate of said expandable compartment.

Optionally, said controller includes a computerized control unit (CPU).

Optionally, said controller includes electronic circuitry.

Optionally, said controller includes a mechanical control device.

Optionally, said controller resides within said device.

Optionally, said controller resides at a location outside the device.

Optionally, the device additionally, includes a parametric sensor, said parametric sensor connected to said fill mechanism in a feedback loop.

Optionally, the parametric sensor provides an output signal pertaining to said expandable compartment

Optionally, the parametric sensor provides an output signal pertaining to a subject in whom the implantable tissue expansion device is implanted.

Optionally, the device includes (o) a surface comprising a plurality of protrusions of at least 5 mm height to prevent shifting of the device after implantation.

In an exemplary case, there is provided an external control device for operation of an implantable tissue expansion device, the external control device comprising:
(a) a signal source designed and configured to convey a signal to a regulator controlling a filling of an expendable compartment of an implantable tissue expansion device; and
(b) a power source capable of supplying power to said signal source to convey said signal.

Optionally, the signal includes a magnetic field.

Optionally, the signal includes an RF wave.

Optionalty, the device additionally includes:
(c) a control module.

Optionally, the control module includes a computerized control unit (CPU).

Optionally, the control module includes electronic circuitry.

Optionally, the device includes
(c) a signal received configured to receive a data signal from a parametric sensor.

Optionally, the parametric sensor senses data pertaining to an expendable compartment of an implantable tissue expansion, device implanted within a subject.

Optionally, the parametric sensors senses data pertaining to a subject in whom the implantable tissue expansion device is implanted

Optionally, the device includes:
(c) a data storage to store data received by the controller from a parametric sensor.

Optionally, the device includes:
(c) a data relay which relays data received by the controller from a parametric sensor to at least one additional data processing device.

Optionally, the device includes:
(c) a data relay which relays data received from at least one data processing device to an implantable tissue expansion device implanted within a body of a subject to control expansion thereof.

Optionally, the device includes: a reminder mechanism capable of issuing a reminder to a subject to operate the device.

Optionally, the device includes a data display to display data.

In an exemplary case, there is provided method of rendering a soft implantable tissue forming device resistant to microbial contamination, the method comprising applying a coating including an antimicrobial agent to at least a portion of the device.

Optionally, the tissue forming device is a tissue expansion device.

Optionally, the tissue forming device is a cosmetic breast implant.

In an exemplary case, there is provided a computer designed and configured to respond to queries concerning performance of implantable tissue expansion devices, the computer comprising:
(a) a memory containing data pertaining to:
   (i) design feature data pertaining to a plurality of implantable tissue expansion devices;
   (ii) operational data pertaining to a plurality of implantable tissue expansion devices employed for treatment in individual subjects; and
   (iii) subject data pertaining to a response of each of said individual subjects in whom one of said plurality of said implantable tissue expansion devices has been implanted.
(b) circuitry configured to receive a query and formulate a response based upon said data stored in said memory.

Optionally, the memory additionally contains:
(iv) compliance data pertaining to a treatment program compliance of each of said individual subjects in whom one of said plurality of said implantable tissue expansion devices has been implanted.

In an exemplary case, there is provided a method of determining a desirable design characteristic of an implantable tissue expansion device, the method comprising analyzing data from a database and identifying at least one design feature which correlates to a favorable subject response.

In an exemplary case, there is provided a method of determining a desirable tissue expansion program for use in conjunction with implantable tissue expansion devices of a given design, the method comprising analyzing data from a database and identifying at least one operational parameter which correlates to a favorable subject response.

In an exemplary case, there is provided a computerized system designed and configured to construct database of implantable tissue expansion device data, the system comprising:
(a) a design feature data acquisition module to acquire and store design feature data pertaining to a plurality of implantable tissue expansion devices;
(b) said plurality of implantable tissue expansion devices, each device designed and configured to acquire and store data on at least one device performance characteristic;
(c) a plurality of subject data parametric sensors designed and configured to acquire and store data on at least one subject response parameter; end
(d) a data relay connectable to each of said tissue expansion devices and parametric sensors for purposes of transferring data to the database.

In an exemplary case, there is provided a method of anchoring a soft implantable medical device configured to modify a shape of a body part, the method comprising providing a plurality of protrusions on a surface of the device so that said protrusions restrict relative motion of the device with respect to a surrounding tissue layer.

Optionally, said protrusions are characterized by a height of 0.5 to 5 mm and adapted to engage a soft tissue.

Optionally, to 500 protrusions are employed.

Optionally, the method is applied to a tissue expansion device and/or a long term cosmetic breast implant.

In an exemplary embodiment of the invention, there is provided a tissue expansion device, the device comprising:
(a) an expandable compartment adapted for implanting in a body of a subject;
(b) a gas source coupled to said compartment; and
(c) at least one regulator adapted to be located within said body and selectively control gas flow from said source to said compartment

### BRIEF DESCRIPTION OF FIGURES

In the Figures, identical structures, elements or parts that appear in more than one Figure are generally labeled with the same numeral in all the Figures in which they appear. Dimensions of components and features shown in the Figures are chosen for convenience and clarity of presentation and are not necessarily shown to scale. The Figures are listed below.
Figs. 1A and 1B illustrate valve mechanisms according to exemplary embodiments of the invention;
Figs. 1C and 1D illustrate membrane based flow control mechanisms according to exemplary embodiments of the invention;
Figs. 2A, 2B, 2C and 2D illustrate actuator mechanism according to exemplary embodiments of the invention;
Fig. 3A illustrates an exemplary implantable device and an exemplary extracorporeal wireless control unit according to the present invention;
Figs. 3B, 3C and 3D illustrate exemplary relative placement of operational components within an exemplary implantable device from side view, top view and oblique angle view respectively;
Fig. 4 is a graph indicating the applied force in grams required to close a valve of the type depicted in figure 1A as a function of gas pressure in PSI;
Fig. 5 illustrates a device according to the present invention implanted in a subject and an option external control unit and an optional remote data monitoring serve;
Fig. 6 is a flow diagram illustrating events associated with preparation and use of a device according to the present invention;

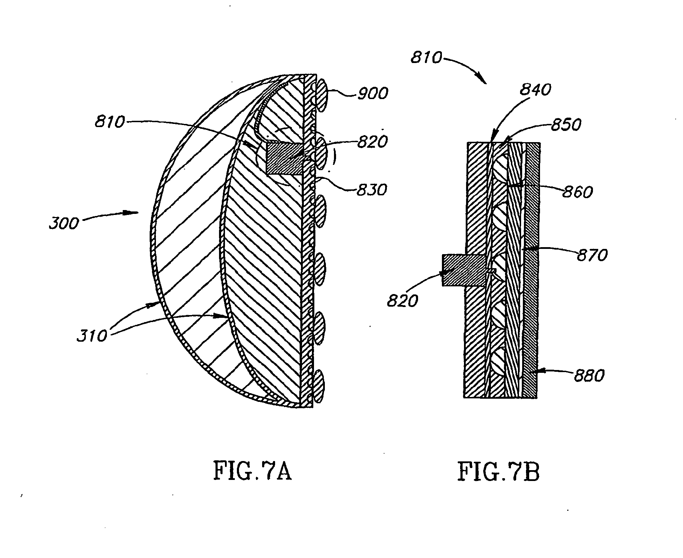

Figs. 7A and 7B illustrate a device according to the present invention which relies upon interstitial fluid for tissue expansion: and
Fig. 8 is a series of plots illustrating the relationship between incremental volumetric additions of gas and resultant internal pressure of compartments with various initial sizes.

### DETAILED DESCRIPTION OF EMBODIMENTS

### General configuration: gas based devices

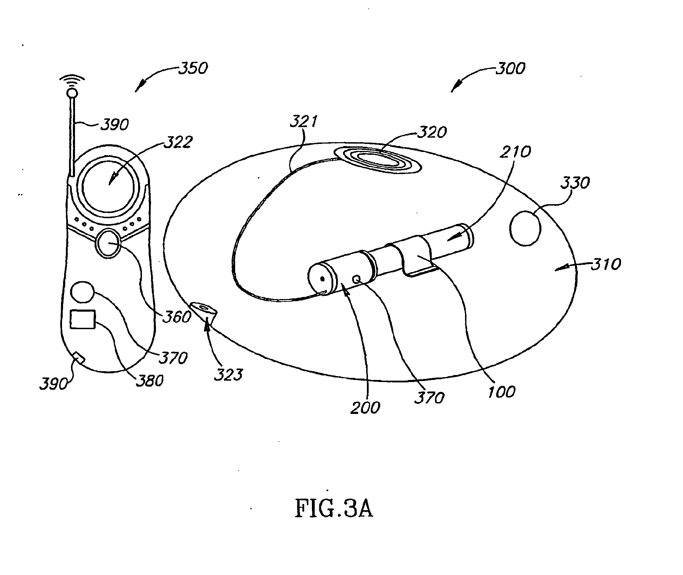

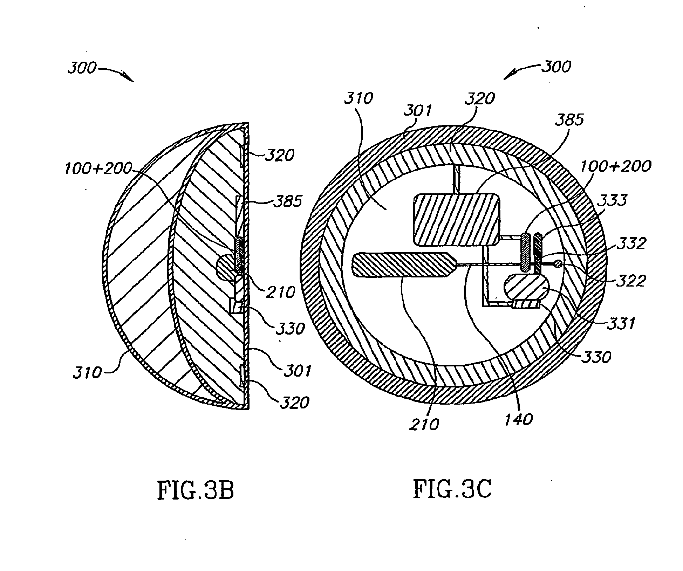

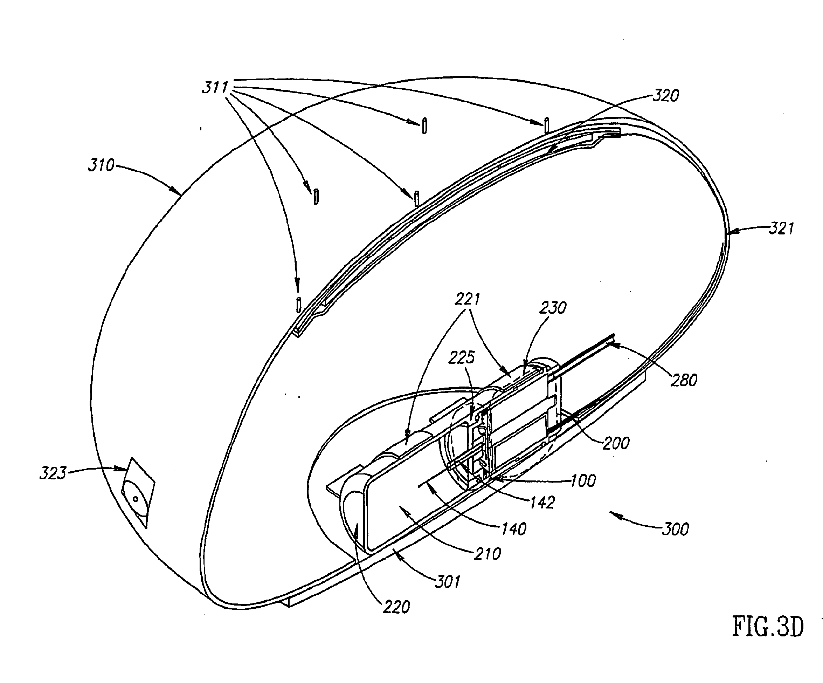

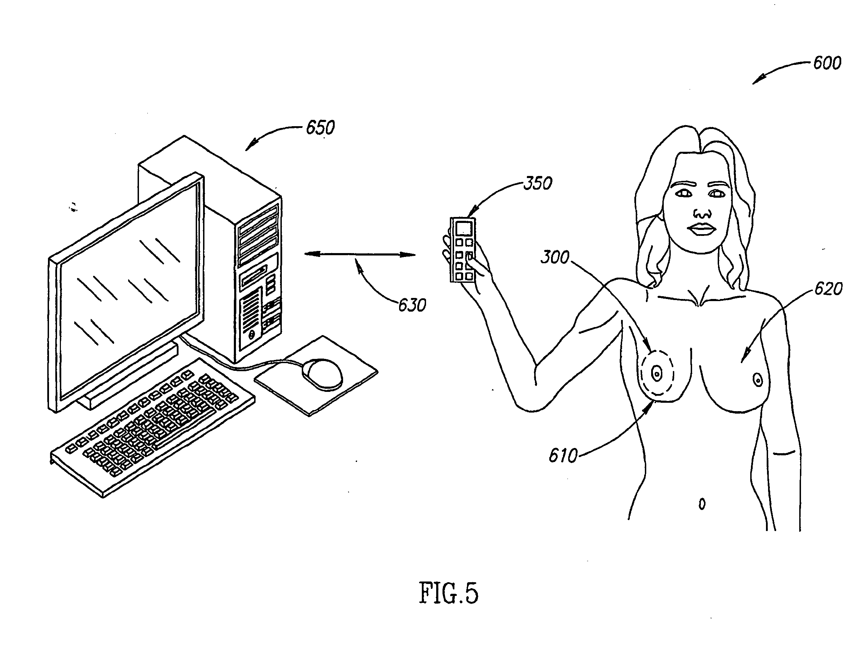

In an exemplary embodiment of the invention, a self contained implantable tissue expansion device 300 (Fig. 3) including an expandable compartment 310 is provided. Device 300 includes a fill source, optionally a gas source 210. In an exemplary embodiment of the invention, (Figs. 3A, 3B, 3C and 3D), device 300 is configured as a breast implant implantable in a breast 610 of a subject 600 (Fig. 5.). This may be undertaken, for example following surgery performed on breast 610 (e.g. tumor resection). Optionally, device 300 expands over a period of time via transfer of gas from gas source 210 to expandable compartment 310. In an exemplary embodiment of the invention, device 300 restores skin and/or muscle tissue of breast 610 to dimensions similar to those of contra-lateral breast 620. Optionally, this facilitates implantation of a long term cosmetic implant in breast 610 so that subject 600 achieves approximate bilateral symmetry with contra-lateral breast 620. Because gas can be packed under pressure in a small volume and later expand to a larger volume at a lower pressure, device 300 may be self-contained. Alternatively or additionally, a device which will eventually assume large proportions may be collapsed and implanted through a small incision.

In an exemplary embodiment of the invention, device 300 relies on a self contained gas source 210. Optionally, source 210 contains a fixed amount of gas. Optionally, a fixed amount of gas makes unwanted over inflation less of a safety concern. In an exemplary embodiment of the invention, the fixed amount of gas in source 210 corresponds to a desired maximum inflation of expandable compartment 310. This makes explosion of compartment 310 as a result of suddon release of the contents of source 210 into compartment 310 unlikely.

Gas source 210 optionally has an internal volume of 1 co to 50 cc, optionally 2 to 10 cc. In an exemplary embodiment of the invention, a compressed gas source 210 has a total internal volume of about 5 ml. Optionally a large tissue expansion may be achieved by providing 2.5 grams of CO2 in a 5 ml internal volume container. This provides about 1200 ml of CO2 at 15 PSI (1 PSI above atmosphere at sea level). Alternatively or additionally, a 0.05 ml CO2 source could provide a final volume of about 12 ml final volume. Optionally, many small gas sources 210 are provided in a single device 300.

In an additional exemplary embodiment of the invention device 300 includes an expandable compartment 310 adapted for implanting in a body of a subject and a gas source 210 coupled to said compartment; and at least one regulator (e.g. 100+200 or 920+930) adapted to be located within said body and selectively control gas flow from said source to said compartment.

In an exemplary embodiment of the invention, the release of gas from source 210 is controlled over a period of time. This contributes to a gradual inflation of compartment 310 which may reduce patient discomfort. Alternatively or additionally, more frequent and/or continuous expansion events may reduce the likelihood of the development of a restricting capsule. Small gradual expansion is hypothesized to result in less capsule formation, i.e. reduced capsule thicikness, than expansion brought about by greater expansive force (pressure). In an exemplary embodiment of the invention, a treatment with device 300 according to the present invention might last 7 to 180 days. Actual treatment time might depend upon factors including, but not limited to, required degree of expansion and/or elasticity of tissue(s) to be expanded and/or growth characteristics of tissue to be expanded and/or subject compliance with treatment.

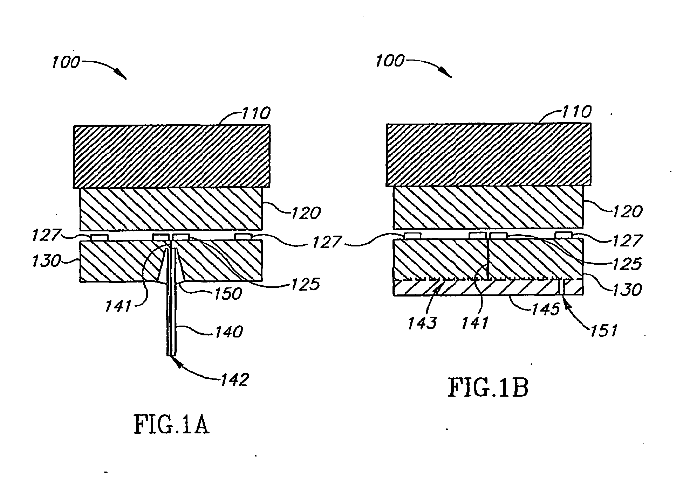

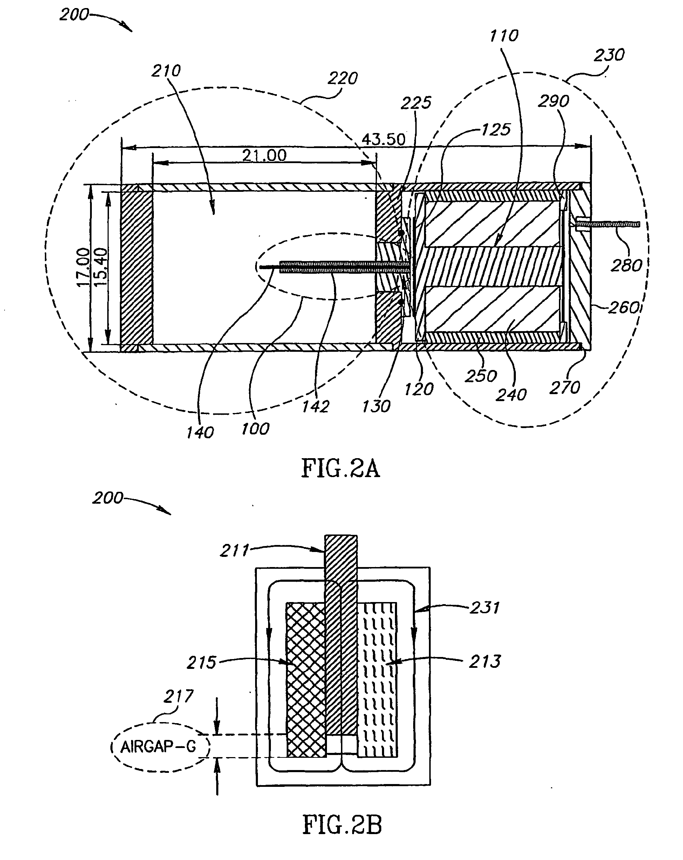

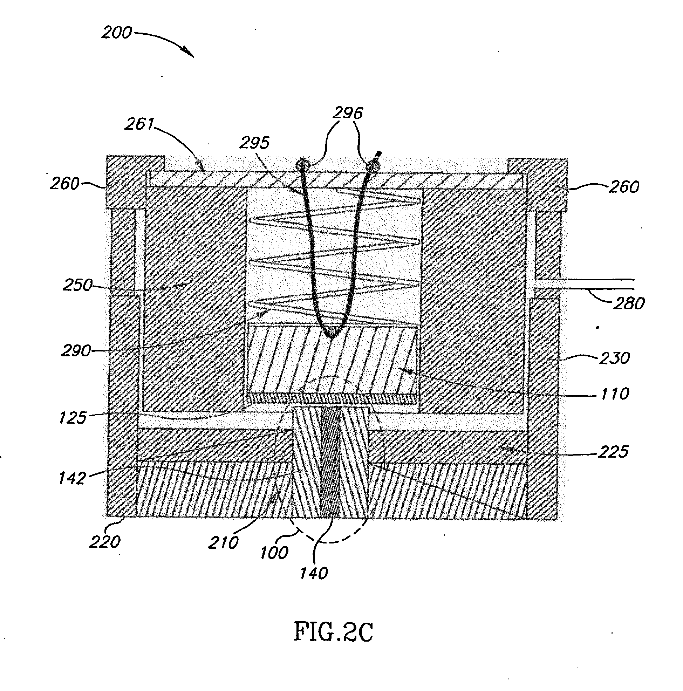

In an exemplary embodiment of the invention, additional control over transfer of gas from source 210 to compartment 310 is achieved by flow restriction. Device 300 optionally includes a valve 100 (Figs. 1A and 1B). Valve 100 may optionally regulate a flow of gas under pressure from gas source 210 into expandable compartment 310. An actuator 200 (Figures 2A, 2B and 2C) may optionally apply additional regulation to valve 100. Actuators 200 are described in greater detail hereinbelow.

In an exemplary embodiment of the invention, a gradual expansion of tissue (e.g. breast 610) is desired. Optionally, adjustment of breast 610 to match contra-lateral breast 620 is desired. Optionally, gradual expansion indicates a period of several weeks, optionally several months, as much as six months or more. Optionally, a low rate of transfer of gas from gas source 210 to expandable compartment 310 is employed. Optionally, valve 100 is characterized by a low flow rate. Optionally, regulation of a flow rate through valve 100 is desired. Optionally, an actuator 200 is included in device 300.

Optionally, device 300 is constructed with consideration of radiation transmission. Optionally, device 300 is constructed primarily of radio transparent materials such as plastic and/or aluminium so that it will not be visible in X-ray images and/or will not interfere with radiation therapy. Alternatively or additionally, at least some parts (e.g. outer shell or gas source 210 or actuator 200) of device 300 are X-ray opaque so that assessment of position via X-ray imaging may be carried out after implantation.

### Exemplary Use Scenario

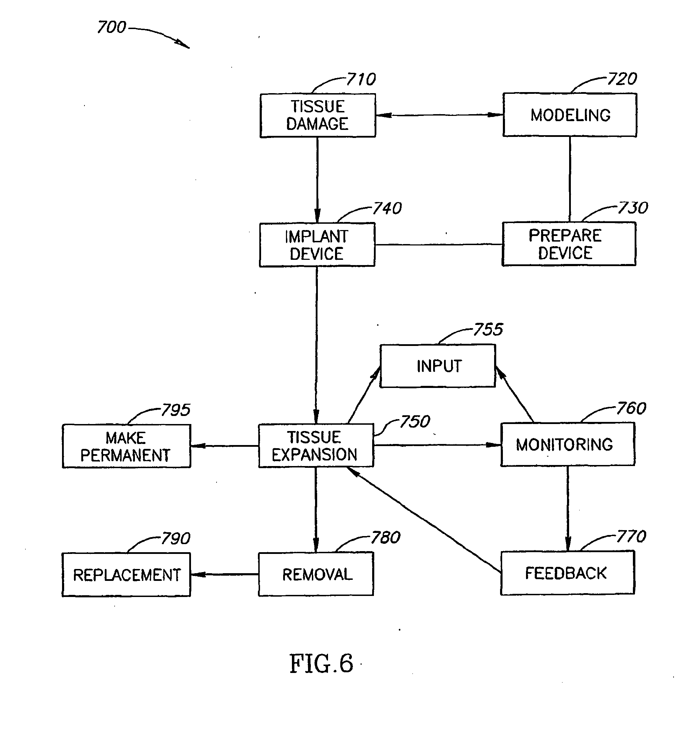

Fig. 6 shows a method 700 of using device 300 to treat a breast cancer patient, in accordance with an exemplary embodiment of the invention. Device 300 is employed as part of a method 700 of repair after a tissue damage event 710 has occurred. Tissue damage 710 may be, for example, a tumor resection, such as a mastectomy. Optionally, modeling 720 of the affected tissue (e.g. breast 610; Fig. 5) is performed prior to tissue damage 710. Optionally, modeling 720 of a matching contralateral tissue (e.g. breast 620) is performed. Device 300 is prepared 730 optionally based on modeling 720. Optionally, device 300 includes thermoplastic or thermosetting sections that are shaped during modeling. Optionally modeling 720 includes calculation of a required incremental inflation volume and/or pressure which may be translated to an amount of a specific inflation gas in grams.

In an exemplary embodiment of the invention, a surgeon may choose a device 300 from among stock configurations. Optionally, stock configurations are chosen based on base circumference. Optionally, stock configurations are designated by bra cup size when fully inflated. Alternative approaches may involve augmentation of the contralateral breast in order to more closely approximate the reconstructed breast

After preparation 730, device 300 is implanted 740. After implantation 740, device 300 expands over a period of time by transfer of gas from source 210 to compartment 310 causing tissue expansion 750. Optionally this process may be regulated or controlled as detailed hereinbelow. Tissue expansion may be subject to input 755, optionally in the form of activation signals. Monitoring 760 of subject and/or device parameters may be used in conjunction with input 755 and/or to provide a feedback loop 770 which functions independently of input 755. Once tissue expansion 750 is judged complete, removal 780 of device 300 may be performed, for example to implant a replacement 790 long term implant. Optionally, device 300 is made permanent, for example by filling with a conventional implant material such as, for example, silicone gel or saline.

In another exemplary embodiment of the invention, tissue expansion device 300 is optionally employed to grow new skin to permit repair of damaged skin tissue 710 at another location. According to this embodiment of the invention, modeling 720 is optionally not pursued because device 300 is intended to disrupt a natural body contour as a means of creating excess skin for subsequent transfer. New skin may be induced to grow by increased tension resulting from expansion of the implanted device as described hereinabove. Optionally, the new skin is harvested and transferred to a new location as an autologous graft. In an exemplary embodiment of the invention, this strategy is employed to effect cosmetic repair. Optionally, the cosmetic repair may be for scar removal, to replace a tattooed area, to replace skin damaged by bums or to ameliorate pigment irregularities. Optionally, skin for transfer is created in a matching body area. For example, repair of a right side of the face might be pursued by implanting a device 300 under the left cheek. Optionally, this might produce skin with similar characteristics to the damaged skin in terms of pigment and/or elasticity and/or hair prevalence and/or hair characteristics. According to these embodiments of the invention, a subject may voluntarily undergo a short term disfigurement in order to overcome long term tissue damage. In an exemplary embodiment of the invention, new skin is molded. Optionally, molding occurs during formation. Optionally, molding occurs during or after transplant. Optionally, molding is in conformation to a form attached to device 300. Optionally, molding is in conformation to a form provided at a transplant site. In an exemplary embodiment of the invention, new skin grown in response to pressure provided by a device 300 is employed to reconstruct an ear.

In an exemplary embodiment of the invention, a device 300 is deployed to create a small degree of expansion, for example to stretch skin in order to affect burn repair in the palm of a hand. A total expansion volume of 10 to 50 ml, optionally 15 to 30 ml, optionally about 20 ml may be sufficient for a clinical application of this type. Because a small device 300 is desired, gas source 210 is optionally scaled down, for example to a total volume of 2 ml or less. Optionally, nitrogen gas under pressure is employed to fill gas source 210 because only a small expansion volume is required. Optionally, a 200 PSI fill pressure for source 210 is sufficient to meet volume constraints. Optionally, an assembly including valve 100 and actuator 200 is scaled down so that it has a generally cylindrical configuration with an OD of 200 to 250 microns. Optionally, a capillary 140 valve 100 with an internal diameter of 25 to 100 microns and an OD of 150 microns is compatible with such a design. Optionally, actuator 200 achieves the desired small volume by employing a Nitinol based mechanism to regulate flow through capillary 140. Alternatively or additionally, a magnet held outside the body but in proximity to device 300 may activate actuator 200 so that gas is released from source 210 into compartment 310. In an exemplary embodiment of the invention, the combined valve and actuator mechanism may be provided in a unit with dimensions of a lead refill for a mechanical pencil (250 micron diameter; 1cm length) and attached to a gas source 210 with a 2 ml overall volume.

### Valves

Exemplary embodiments of valves (Figs. 1A and 1B) and actuators 200 (Figs. 2A, 2B and 2C) are presented as exemplary embodiments of flow regulation to provide inflation over a period or weeks is feasible, although not all embodiments of device 300 require a valve 100 and/or an actuator 200.

One way to restrict a flow rate of gas is to force it to flow through a narrow outlet, optionally after it passes through an elongate path. Figs. 1A and 1B show exemplary configurations of narrow orifice valves. Optionally, valves 100 of this general type may be employed in a device 300. Both of these drawings are oriented so that gas source 210 (not shown) is below valve 100 and gas flows through a first block 130. Optionally, block 130 is constructed of silicon. Optionally, block 130 is constructed of metal, such as stainless steeL Silicon parts positioned between the high pressure and low pressure compartments of the valve may optionally serve to restrict and regulate the gas flow including stopping flow altogether. A seal 125 is positioned at the exit point from gas source 210. This seal 125 may be formed of elastomeric or rigid materials as seal-pressure and gas parity conditions permit For example, elastomeric materials may be preferred if the source 210 gas contains some small particles on the order of 0.5 µm or larger. Gases of higher cleanliness level may allow use of metallic or other rigid materials. If this seal 125 is open, gas flows through valve 100 and may pass into expandable compartment 310 (not shown). Optionally, valve 100 is characterized a flow rate when open. Flow rate may vary according to a gas pressure in source 210 and/or valve characteristics.

Both of these drawings (Figs. 1A and 1B) are oriented so that a piston 110 is positioned above seal 125 and exerts a downward force on an upper block 120, also optionally silicon, so that it descends and presses on seal 125. Pressure on seal 125 prevents further flow of gas through narrow orifice 141 of valve 100. Elastomeric seal 125, optionally provided as a ring, is pressed between blocks 130 and 120. This prevents further transfer of gas. Additional elastomer pads 127 are optionally provided. Additional pads 127, optionally provided as a ring, may assure that blocks 120 and 130 remain parallel so that seal 125 closes efficiently when piston 110 applies force. Elastomeric as used herein refers to any deformable polymer such as, for example, Viton, silicone or any of a wide variety of rubbers, plasticized polymers or other moderate elastic modulus polymer materials. The basic configuration of valve 100 includes a narrow orifice 141. Optionally, a seal (e.g. elastomer 125) which can be engaged or disengaged is also provided.

In Fig. 1A, a capillary tube 140 supplies the narrow orifice. In the figure tube 140 is installed in an epoxy plug 150 in lower block 130. An inner lumen of capillary tube 140 is in fluid communication with a hole in seal 125 through a channel 141 etched in silicon block 130. Optionally, channel 141 has a diameter of approximately 50 microns. Optionally, in designing a valve 100 of this type, there is a compromise between choosing materials which are soft enough to provide a desired degree of deformation and stiff enough that an end of the capillary will not become exposed and/or break off and/or tear other components.

In Fig. 1B, channel 141 transects block 130 and is in fluid communication with a tortuous path etched on a lower surface of block 130 and covered by a glass cover plate 145. Embodiments depicted in Figs. 1A and 1B each employ long narrow path to provide flow resistance and reduce flow rate. The tortuous path of Fig. 1B additionally requires the gas to change directions. Optionally, this creates additional resistance and/or flow reduction and/or provides particle filtration. In this exemplary configuration, gas enters tortuous path 143 of valve 100 through a gas entry port 151 cut in glass plate 145.

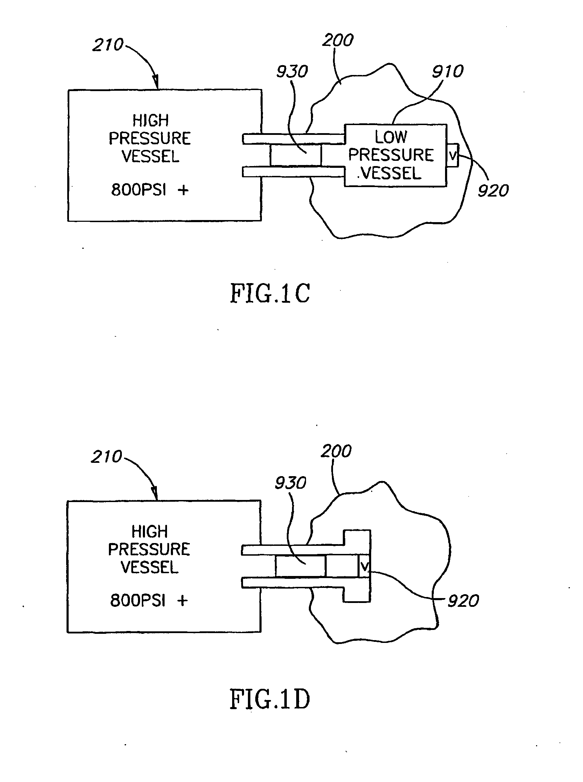

Fig. 1C illustrates a mechanical flow control device which integrates valve and actuation functions by employing a membrane 930 with a characteristic diffusion rate with respect to high pressure gas in source 210. Membrane 930 is selected so as to allow for a continuous steady release of gas based upon the permeation of a gas through the material. This rate controlling membrane is selected from a group of materials based upon the permeation rate of the gas in source 210 and the thickness of membrane 930 that is employed to provide for a constant slow release of gas from the reservoir. For example, if a relatively high permeation rate is desired to deliver oxygen a material such as polypropylene with a permeation rate of 82.07 cm³ mm/ m² day atm may be employed, for an intermediate rate one could employ nylon 6,6 for the membrane material which shows a transmission rate of 2.027 cm³ mm/ m² day atm. A low rate could be obtained using a material such as ethylene vinyl alcohol with a permeation rate of 0.0041 cm³ mm / m² day atm. One of ordinary skill in the art will be capable of selecting an appropriate membrane material, cross sectional area and thickness of the membrane in order to achieve the desired gas flow rate for a specific embodiment of device 300. In the depicted exemplary embodiment, gas flows through membrane 930 to an accumulation chamber 910 characterized by a low pressure. If gas flow were allowed to continue, the pressure in chamber 910 would eventually equilibrate with that in source 210 and flow across membrane 930 would cease. In order to assure that flow continues, a release valve 920 with a constant pressure set point below the pressure in source 210 is installed on chamber 910. When gas pressure in chamber 910 reaches the set point of valve 920, the valve opens and gas from chamber 910 is released into compartment 310. This arrangement results in periodic release of similar amounts of gas. Periodicity and amount of gas release at each opening of valve 920 will be related a pressure in source 210, characteristics of membrane 930, volume of chamber 910 and set point of valve 920. Optionally, valve 920 is installed in a wall of 910 using threaded connections. In an exemplary embodiment of the invention, this permits a set point of valve 920 to be altered by changing the valve. According to this exemplary embodiment of the invention, pressure in source 210 supplies all the required power for expansion.

A gas release mechanism of the type depicted in Fig. 1C may also be used in other applications, for example delivery of a material stored chamber 910 and/or gas source 210. Optionally, the material may be a medication. Because the gas release mechanism can be scaled down in size, it may be configured for use as a metered dose device, optionally for controlled release of medication over a period of weeks to months. Optionally, the gas release mechanism is coupled to a metered delivery system and/or a valve and/or a flow constriction device and/or actuator (not pictured). Alternatively or additionally, a gas release mechanism for therapy where the active agent is the gas itself (CO2 as in Capnia, NO, 02, etc; www.capnia.com/) or dispersed in the gas as described above. Previously available liquid form delivery system of an active agent in an implantable device (e.g. osmotically driven Alza implants called Durect; www.alza.com) did not permit regulation. A gas release mechanism as depicted may power the elution of the active agent from device 300 and impart the ability to turn the device off and on and meter it based on modulation of the duty cycle of the on/off cycles.

Fig. 1D shows a similar arrangement with no chamber 910 between membrane 930 and valve 920. Optionally, this arrangement provides more frequent transfer of gas to compartment 310 and/or release of smaller amounts of gas. In an exemplary embodiment of the invention, this arrangement approaches continuous release, optionally at a very low flow rate.

In an exemplary embodiment of the invention, gas source 210 is filled with gas under sufficient pressure so that a portion of the gas condenses into the liquid phase. Optionally, less than half of a volume of source 210 is filled with liquefied gas. Optionally, capillary 140 is of a length so that distal end of tube 142 and/or capillary 140 does not contact this liquid gas even if gas source 210 is inverted. In an exemplary embodiment of the invention, carbon dioxide gas is employed in conjunction with a tortuous path valve of the type shown in figure 1B. Carbon dioxide remains in the dense gas phase (above its critical point) at body temperature, even under high pressure. Other gases which liquefy under these conditions maybe less suited to use with a tortuous path valve. Optionally, gas in a liquid phase might enter gas entry port 151. Optionally, this possibility may be prevented by employing a known liquid flow preventing valve.

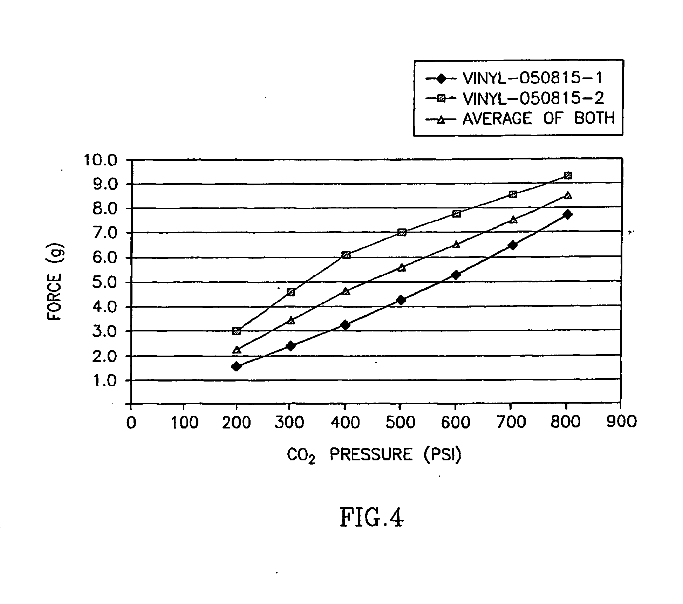

Fig. 4 is a graph of required applied force to close a valve 100 as a function of gas pressure in source 210. The graph shows that a capillary valve may be closed with an applied force of less than 10 grams even when the gas pressure in gas source 210 is in hundreds of PSI. This means that an actuator 200 capable of supplying only a small force may be effectively employed to control flow through valve 100. Because only a small force is required from the actuator, it is possible to employ a low power mechanism. Optionally, 100 to 500 mW, optionally 200 to 250 mW of power is sufficient to drive actuator 200. This optionally contributes to a reduced size of device 300 by allowing use of a small power source (e.g. battery). Optionally, a small battery capable of a low power output over a prolonged period of time may be employed. Optionally, the small battery facilitates extended intra- body deployment. Alternatively or additionally, an external power source may be employed to power an actuator with out a physical percutaneous link as described in greater detail hereinbelow. Supplying a power source outside of device 300 optionally reduces the size of device 300. The basic operating principle of valve 100 includes application of a seal, optionally elastomeric, to narrow orifice 141. The presented results are from a test in which a 200 micron thick vinyl sheet was employed to seal a 50 micron internal diameter and 150 micron external diameter capillary in the capillary only based design. In an exemplary embodiment of the invention, valve 100 is capable of shutting off a flow from a gas source 210 with an internal pressure of 1300 PSL The exact amount of force required to achieve this may vary with diameter of orifice 141 and/or valve configuration (e.g. capillary tube or tortuous channel) and/or elastomer and/or applied gas pressure. Optionally, additional pads 127 are provided as an elastomeric o-ring as a lithographically determined structure on the silicon design (e.g. 120 or 130, or the vinyl or other elastomer sheet).

The narrow orifice valve may be configured to have an average flow rate of approximately 5 ml/second when the gas pressures in source 210 are in the range of 200-1300 PSI. In an exemplary embodiment of the invention, valve 100 is configured so that a 5mL/s average flow rat results from a gas source 210 with a an initial charge of 800 PSL Although Poiseuille's Law describes liquid or gas flow in a narrow channel, design of valves 100 was refined empirically because available formulae did not seem to account for all relevant variables.

In an exemplary embodiment of the invention, a valve in an implantable tissue expansion device 300 could discharge its entire contents of approximately 1200 ml (2.5 grams of CO₂) in slightly more than 4 minutes. In practice, actual flow rate might vary from 1 to 20 ml/second during most of the transfer of gas from source 210 to compartment 310. In an exemplary embodiment of the invention, a source 210 with a desired amount of gas for deployment in a short term is discharged in an open loop arrangement

Optionally, several such sources are employed, either successively or concurrently or a combination thereof. Optionally, sources 210 might be discharged at a fixed interval, for example once every 8 hours. Release of gas from sources 210 might optionally be accomplished by a single use actuator or by rupture of a seal or by rupture of a source 210. Actuation will be discussed hereinbelow. Rupture might be accomplished, for example, by mechanical or electrical means. Optionally, a microprocessor might be employed. Optionally, a series of open loops are organized into a program. Optionally, the program includes a feedback loop. In an exemplary embodiment of the invention, a series of open loop discharges, optionally through valves 100, are implemented over a course of several weeks. Optionally, each open loop terminates automatically when an amount of gas in a source 210 is discharged. Optionally, the amount of compressed gas (maybe in the liquid or gas or supercritical state depending on specific gas, reservoir volume and amount stored gas in this form) is between 1 and 100 ml per source 210. In some embodiments, an average inflation rate of 5 ml/second is too high and additional regulation may be supplied.

### Actuators

One way to provide additional regulation is to employ an actuator 200. Actuators 200 may be powered by power sources of various types. Power may be employed, for example, to apply and/or remove a force from seals 125 of a valve 100 as described hereinabove. Optionally, power is supplied from a power storage device such as, for example, a battery. Alternatively or additionally, power may be supplied from an external source, for example via matched RF coils as detailed hereinbelow. Optionally, actuation is regulated by one or more feedback loops as described hereinbelow. In general, a required power input will vary with the required force per unit travel of a moving part (e.g. piston) of the actuator. Therefore, a short operational distance may optionally reduce a requirement for power and/or permit high frequency operation using a limited power input. Narrow orifice valves are optionally characterized by short operational distances. In an exemplary embodiment of the invention, actuators 200 with short operational distances are employed. Optionally, the actuators may be normally open or normally closed. Optionally, a flow rate of valve 100 which is nominally too high may permit use of an actuator with a duty cycle having a desired closed to open ratio.

### SOLENOID ACTUATORS

In an exemplary embodiment of device 300, a solenoid actuator 200 may be employed (Fig. 2A). In the pictured embodiment, an actuation mechanism is contained in an actuator housing 230 which optionally shares a common wall 225 with housing 220 of gas source 210. Common wall 225 serves to reduce overall size and/or weight of device 300 without compromising structural integrity. Valve 100 employs a capillary tube 140, surrounded by a polyether ether ketone (PEEK) tube 142. Optionally, PEEK tube 142 provides mechanical support for capillary 100. Optionally, tubing 142 may be constructed of a metal, another polymer or other material with the desired rigidity. Capillary tube 140 is fitted in block 130 (optionally silicon). In the pictured configuration, elastomer seal 125 is positioned on second block 120 (optionally silicon) which is mounted on piston 110. When valve 100 is open, gas from source 210 exits tube 140 and passes through tube 280 to expandable compartment 310 (not shown). The actuation mechanism includes a solenoid coil 240 in a threaded solenoid housing 250. Op threading of housing 250 permits exact positioning and/or an effective gas seal for the housing so they all gas discharged from source 210 is routed to compartment 310. A flat spring 290 is operable to cause piston 110 to descend. This causes seal 125 to close the end of capillary tube 140. A threaded cap 260 with a low pressure seal 270 is optionally provided.

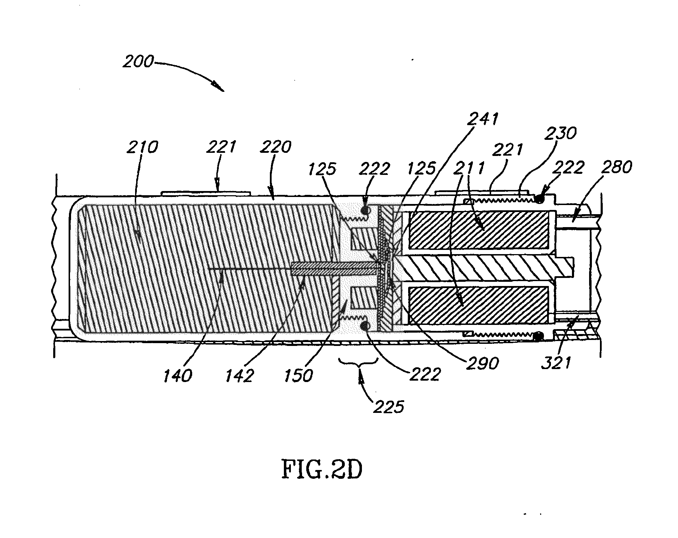

An additional exemplary configuration of a solenoid actuator (Fig. 2D) relies upon a spring, optionally a flat spring 290 to force seal 125 against capillary 140 to shut off a flow of gas. Seal 125 is mounted on a plate 241 at least partially constructed of a magnetic metal. Application of an electric current to solenoid coil 240 magnetizes core 211. Core 211 generates a magnetic field with sufficient force to overcome the elastic strength of spring 290 and pull plate 241 bearing seal 125 away from capillary tube 140. This results in a flow of gas through tube 140 and outwards through exit port 280 to expandable compartment 310. Optionally, opposite configurations in which electric current is applied to coil 240 to close the valve and the valve is open in the absence of applied current are also feasible. Optionally, the configuration of actuator 200 is selected in accord with the desired duty cycle. Optionally, power is conserved by choosing an actuator configuration in which a desired operational state (i.e. valve open or valve closed) is achieved for a majority of time with no applied electric current Optionally, O-rings 222 are used to seal housing 220 and/or 230.

### FLAT SPRING ACTUATOR

Optionally, spring 290 is a Nitinol spring which contracts and expands as current is applied and removed. Current may be applied from a power source, such as a battery. Optionally, the power source is external to device 300 as described in greater detail hereinbelow. Optionally, current is applied cyclically, according to a program. Optionally, the program is implemented by a computerized controller. Current flow through spring 290 causes heating which results in a conformational change. Expansion and contraction of the spring could serve to move piston 110 instead of, or in addition to, the solenoid mechanism. Optionally, spring 290 does not have elastic characteristics in the sense of a conventional spring, but expands and contracts in response to the application and/or removal of an electric current

### ELECTROMAGNETIC ACTUATOR

In another exemplary embodiment of the invention, an electromagnetic actuator 200 is employed (Fig. 2B). Actuator 200 includes a ferromagnetic metal frame 231 (e.g. soft iron), a moveable plunger 211, an electric coil with current in a positive direction 213 on one side and a negative direction 215 on the other side. Current flow in the coil causes displacement of plunger 211 through distance 217. Reversal of the current direction coil (i.e. 213 negative and 215 positive) will cause plunger 211 to move in the opposite direct. A distance 217 of 0.075 mm is sufficient for actuation of valve 100 with a design of this type.

### NITINOL WIRE ACTUATOR

In another exemplary embodiment of the invention, a nitinol wire actuator 200 may be employed (Fig. 2C) instead of a solenoid actuator. Nitinol actuator 200 is shown positioned above gas source 210 contained in gas source housing 220 which is generally as described for figure 2A. Valve 100 in the form of capillary channel 140, elastomeric seal 125 and capillary wall 142 is pictured. In the pictured embodiment, seal 125 is mounted on piston 110. Motion of piston to close seal 125 against capillary 140 is supplied by energy of spring 290 installed above piston 110 in housing 250. Housing 250 is installed in actuator housing 230 and retained therein by insulated cover 261 and threaded ring 270. Application of electric current to Nitinol wire 295 causes wire 295 to contract. This exerts a force on spring 290 and causes piston 110 and seal 125 to move away from capillary 140 which releases gas into actuator housing 230. Gas flows outward through exit port 280 to expandable compartment 310 (not shown). When the electric current is shut off, nitinol wire 295 cools off and elongates releasing spring 290 which pushes piston 110 and seal 125 against capillary 140 closing valve 100. According to an alternative embodiment of the invention, spring 290 pulls piston 110 away from capillary 140 in a relaxed state and current through Nitinol wire 295 causes the spring to extend, pushing piston 110 downwards. A displacement of 150 microns opens valve 100 with an actuator of this type. A displacement of 200 microns may optionally be achieved with a power input of 100 mW using a Flexinol Nitinol wire of 0.002-0.005 inch diameter. In an exemplary embodiment of the invention, a valve 100 which is normally closed is employed in an application which requires a low flow rate. In the absence of a power input, valve 100 remains closed and no gas flow is permitted (pictured embodiment). In this type of embodiment, a power input is only required when the valve must be opened. Because the low flow rate may be achieved by leaving the valve closed most of the time, power is optionally conserved. Optionally, a 50 micron displacement is required to open valve 100 and power sufficient for a 200 micron displacement is applied to nitinol wire 295. In an exemplary embodiment of the invention, a short burst of power, for example 100mW for 1 second is applied to wire 295. After a first time increment which is less than 1 second the valve is opened by a 150 micron displacement of piston 110. After the 1 second power burst ends, it takes an additional increment of time for wire 295 to expand to the point that valve 100 is closed by the return of piston 110 past the 150 micron activation distance. This means that valve 100 may opened for a time longer than the duration of the applied power input. Optionally, a Nitinol flat sheet acting as spring and moving element in response to heat may be employed. Optionally, Nitinol may be replaced by other heat deformable materials with desired thermal expansion characteristics, such as a bi-metal coil. Heat may optionally be generated by running electrical current through the heat deformable material and/or by installing a heating element in close proximity to it.

### MAGNETIC ACTUATOR

In an exemplary embodiment of the invention, spring 290 (Fig. 2C) naturally pushes piston 110 downwards so that seal 125 closes valve 100. If piston 110 is constructed of a ferromagnetic material, positioning of a magnet at the position indicated by connectors 296 could overcome the force of spring 290, moving piston 110 and opening valve 100. According to this embodiment, valve 100 remains open until the magnet is removed. This magnetic actuator requires no power input beyond that supplied by the magnet. In an exemplary embodiment of the invention, a subject initiates a flow of gas from source 210 to compartment 310 by bringing a magnet into proximity with device 3oo to activate valve 100 through actuator 200.

Optionally, valve configurations having features of valves 100 of figures 1A and/or 1B are employed in conjunction with actuator 200. Optionally, alternate valve configurations are employed.

### Valve/Actuator apabilities

Valves of the general type depicted in Figs. 1A-1D in conjunction with actuators of the general type depicted in Figs. 2A-2D are capable of stopping 150 PSI to 1500 PSI of gas or liquid. These valves are reliable enough for medical implantation and dozens of operational cycles, requiring less then 500 mW, optionally less then 150 mW, of power to activate, consisting of as little as 1 to 4 mm³ (orifice and/or membrane diffusion barrier as flow restrictor, Nitinol actuator) and as much as 10 to 20 cm³ (electromagnet based design). Optionally, a size of 4 mm³ to 10 cm³, optionally 10 mm³ to 5 cm³, optionally 100 mm³ to 5 cm³ optionally 2 cm³ to 5 cm³.

In an exemplary embodiment of the invention, valve 100 and actuator 200 function together as a regulator to regulate a flow of gas source 210 into a low pressure compartment 310. The regulator includes a narrow outlet 141 adapted for connection to gas source 210 with a pressure of at least 20 PSI and as much as 200, or 300 or 500 or 1000 or 1200 or 1500 PSI or more.

The mechanism relies upon a seal 125 (optionally elastomeric) applicable to outlet 141 to stop the flow of gas source 210 into a low pressure compartment 310. Seal 125 is alternately applied/removed to outlet 141 by an actuatable component 110 capable of selectively applying and removing a force to seal 125 thereby selectively preventing and allowing gas flow.

In an exemplary embodiment of the invention, a force applied through seal 125 is less than 10 gram-force. In an exemplary embodiment of the invention, actuatable component 110 requires a power input of less than 500 mW, optionally lee than 300 mW, optionally less than 200 mW, optionally 150 mW or less. In an exemplary embodiment of the invention, a low power input producing a small force is sufficient to prevent a flow of gas from a source 210 with an internal pressure in the range of 150 to 1500 PSI or more. Optionally, the outlet has a cross sectional area less than 0.05 mm², optionally less than 0.03 mm², optionally less than less than 0.01 mm².

In an exemplary embodiment of the invention, a regulation mechanism to regulate a flow of gas from a pressurized gas source into a low pressure area relies upon a membrane 930 which restricts a gas flow rate from the gas source with a pressure of at least 20 PSI; and a valve 920 with a pressure set point lower than a pressure in the gas source and higher than a pressure in the low pressure area. This embodiment is optionally powered only by the pressure of source 210. Optionally, a switch to turn the flow of gas on and off is included.

### Expendable compartments: construction

In an exemplary embodiment of the invention, expandable compartment 310 includes an elastic balloon and/or an inelastic shell.

Optionally, an elastic balloon develops an internal pressure at a volume near its initial volume and maintains that volume throughout inflation. An elastic balloon may be, for example, a silicon balloon such as a dip molded Silicon balloon of the type manufactured by Specialty Silicon Products, Inc. (Ballston Spa, New York, USA). Alternatively or additionally, a gas impermeable elastomer such as butyl rubber and/or poly (isobutylene) may be employed in formation of an elastic balloon.

Optionally, an inelastic shell provides puncture protection and or contains gas released by an inadvertent rupture of an elastic balloon. This safety feature is operative whether the balloon is inside the shell or outside the shell. Alternatively or additionally, an inelastic shell may release gas slowly in case of puncture. An inelastic shell may optionally include film laminates such as, for example, metalized Mylar (PET) (e.g. MC2-100; DuPont Teijin Films Hopewell, VA, USA) or metallized nylon or other metallized polymer films that may act as gas diffusion barriers, or a laminate of polypropylene, polyethylene or nylon as an outer skin with an inner gas barrier of poly(vinylidene chloride) and a polyethylene inner layer used for thermally bonding the film made by Dow Chemical Co (for example, XUR-1689, Midland, MI, USA) are suitable for use in the invention. In an exemplary embodiment of the invention, the inelastic shell is shaped by folding, optionally pleating or accordion folding.

Optionally, the inelastic shell is installed inside the elastic balloon so that accordion like unfolding of the inner shell is less apparent from outside. Optionally, the elastic balloon is soft and thick to facilitate this feature. In an exemplary embodiment of the invention, a biologically inert elastic balloon (e.g. a silicon rubber balloon) contains an inelastic shell within it so that surrounding tissue contacts only biologically inert materials. Optionally, one layer controls gas diffusion and/or imparts a desired shape. Optionally, one layer regulates expansion by providing a resistive force.

Optionally, expandable compartment 310 provides a natural body contour and/or natural feeL This may be accomplished, for example, by using a target tissue to model compartment 310. For example, a breast 610 (Fig. 5) prior to tumor resection, and/or a contralateral breast 620 might be measured and/or cast to provide appropriate dimensions and/or aspect rations for a breast implant device 300.

Alternatively or additionally, an inelastic shell may provide puncture and/or leak protection. In an exemplary embodiment of the invention, rigid components of device 300 (e.g. gas source 210) are stored within compartment 310 which is optionally an elastic balloon. It is conceivable that an excessive force applied to a body part might cause a gas source 210 within an elastic balloon to rupture and/or puncture the balloon.

Alternatively or additionally, an inelastic shell may be employed to prevent inadvertent overexpansion by providing a finite limit to expansion of an elastic balloon. The limit may be, for example a spatial configuration and/or volume of a contralateial organ (e.g. breast 620).

### Expendable comportments: integrity

Optionally, total gas leakage from compartment 310 is less than 5 ml/day, optionally less than 1 ml/day optionally about 0.11 ml/day. In an exemplary embodiment of the invention, the gas is selected to provide a desired leakage rate in combination with materials used to construct compartment 310. Desired rates may be achieved, for example, with film laminates as described hereinabove. Optionally an inner rubber balloon such as one made with butyl rubber further reduces leakage rates. Optionally the use of an inner coating on the outer shell reduces leakage rates. Optionally, a gas which is readily absorbed by the surrounding body tissue is employed so that leakage does not require use of a percutaneous release port. Sealing of a Mylar shell of this type may be accomplished, for example, by application of heat and pressure using a commercially available heat sealer such as the one suitable for tray sealing for medical device packaging. For example, a 5 mm seal may be created by applying a 150 degree centigrade heating element with a pressure of 40 PSI for 1 second. For industrial production, heating elements may be specially shaped to produce implants with desired configurations. Additionally seals may be prepared by the use of an appropriate adhesive to allow for bonding of the sheets. Alternatively or additionally, inelastic sheets of different sizes and/or shapes may be bonded together to preform the implantable device. Optionally, a desired leakage rate is achieved by device 300 by construction using materials with known leakage or permeation characteristics. This may be accomplished, for example, by employing materials with desired permeability and/or diffusion characteristics in construction of compartment 310. Alternatively or additionally, a pressure release valve may be incorporated into compartment 310 to prevent undesired over inflation. In an exemplary embodiment of the invention, carbon dioxide is employed for inflation of compartment 310 and small amounts of excess gas may be safely vented from compartment 310 within the body. Alternatively or additionally, gas may be vented outside the body through a transcutaneous port 323. Alternatively or additionally, a needle may be provided, optionally adjacent to device 300 in a subject operable safety housing, to permit rapid deflation of compartment 310. In an exemplary embodiment of the invention, excess gas from compartment 310 is release through a port 323 into a closed compartment containing a chemical absorbent This eliminates the gas without venting into the body and without use of a percutaneous port

Alternatively or additionally, a semi-rigid or rigid backing 301 may be included within, or bonded to, compartment 310 (Fig 3B and 3C). Backing 301 may, for example, provide an orientation or anchor within the body. Alternatively or additionally, backing 301 may direct expansion of compartment 310 in a desired direction and/or provide a fixed aspect. In an exemplary embodiment of the invention, a breast expansion device 300 includes a semirigid siliconized rubber disc 301 which can be deployed between skin and muscle and/or among or between muscle fiber bundles and/or beneath a muscle layer (e.g. pectoral muscles in breast reconstruction). This optionally prevents unwanted pressure on the ribs. Optionally, operative components of the device are mounted on rigid disc 301 (Fig. 3B). Optionally, the inelastic shell encloses operational components of device 300 such as actuator 200 and/or gas source 210 which are outside of expandable compartment 310 in the form of an elastic balloon. Optionally, a pressure sensitive switch between an inelastic shell and an elastic balloon provide is provided. Optionally the switch closes actuator 200 when pressure is applied.

### General design considerations

In an exemplary embodiment of the invention, a desired size and conformation of device 300 after expansion is known in advance. Because the total desired inflation volume of expandable compartment 310 is known, source 210 of device 300 configured to provide the desired volume by controlling an amount of gas loaded therein. Gas source 210 may be filled, for example, by using carbon dioxide at 800 PSI (room temperature) flowing through a 2 micron particulate filter into capillary tube 140 surrounded by PEEK tube 142. Source 210 is purged twice with pressurized gas and placed in an ice bath. Carbon dioxide gas condenses into source 210 at a rate of about 0.02 g/s so that a 2.5 gram charge of CO2 may be achieved in just over 4 minutes. The exact amount of charge may optionally be determined by monitoring the extra weight of source 210. Once source 210 is filled, valve 100 may be attached. Attachment may be, for example, vial mated sets of threads on source 210 and valve 100. Optionally, a low loss "normally closed" valve 100 is employed and source 210 may be filled days, or even weeks, before deployment in device 300. Optionally, an additional seal is employed to reduce gas loss through valve 100 during storage. Optionally, sources 210 with desired increments of gas fill are prepared commercially and supplied as components for installation in device 300. Optionally, installation in device 300 is performed prior to implantation. Optionally, devices 300 are produce with source 210 installed. In exemplary embodiments in which source 210 is located within compartment 310, installation may be at time of manufacture of device 300. In an exemplary embodiment of the invention, device 300 has a pre-implantation shelf life of at least 30, optionally at least 90, optionally at least 180, optionally at least 365 days or more.

As depicted in Figs. 3A-3D gas source 210 and/or valve 100 and/or actuator 200 may optionally be contained within expandable compartment 310. This protects these components and/or gives a natural contour to the body of the subject by concealing their rigid outlines. Alternatively or additionally, this configuration may make the subject less aware of the presence of more rigid components of device 300 by using expandable compartment 310 as a cushion. For example, a subject attempting to grow new skin on their face (e.g. for autologous graft) may be fitted with a device 300 in their right cheek. If source 210 and/or valve 100 and/or actuator 200 were installed adjacent to compartment 310, the subject might feel these components, for example while trying to sleep on the right side. By installing these components inside compartment 310, they are hidden within an inflatable cushion and the subject becomes less aware of their presence. Optionally, inflatable cushion/compartment 310 permits the subject to fall asleep more easily. Similar considerations apply for breast expansion embodiments.

Alternatively or additionally, subject awareness of these components is reduced by making them small as detailed hereinabove. Although a maximum expansion size of about 1200 ml is sufficient for most tissue expansion applications, devices 300 employing gas sources 210 with the power to provide more than 1200 ml of expansion volume are within the scope of the invention. A variety of gases including, are suitable for use in the context of gas source 210. The choice of gas may optionally depend upon the intended use for device 300.

The gas, or mixture of gases, may optionally be stored in source 210 in liquid, gas or supercritical state. Optionally, gas source 210 contains 50% by volume of gas in a liquid state.

In an exemplary embodiment of the invention, tissue expansion applications which require small expansion volumes, sufficient filling of source 210 may be achieved with a gas that romains in the gas phase in source 210. In an exemplary embodiments of the invention, a face expander 300 employs a small amount of gas. For these types of small expansion applications, gases that are both compressible and biologically safe might be employed. Examples of compressible biologically safe gases include, but are not limited to, oxygen, nitrogen, argon, xenon and neon etc.

In an exemplary embodiment of the invention, tissue expansion applications which require large expansion volumes (e.g. breast reconstruction) the gas in source 210 is optionally liquefied to store sufficient quantities in a smaller volume. Optionally, carbon dioxide, sulfur hexafluoride, and Freons ate suitable for use in this context. Many freons are nontoxic and all are non flammable.

Optionally, device 300 includes a power source to drive actuator 200 and/or a chemical or electrochemical reaction. The power source may be, for example, a battery. In an exemplary embodiment of the invention, power source 370 is looatcd in an external control unit 350 as described in greater detail hereinbelow. Alternatively or additionally, power source 370 may be provided as part of actuator 200. Device 300 derives most of the power required for expansion from the pressure differential between compartment 310 and source 210. Additional power, optionally electric power, serves only to facilitate a transfer of gas from source 210 to compartment 310.

In an exemplary embodiment of the invention, expansion of the expandable compartment is via an open-loop expansion mechanism In which gas is continuously release into expendable compartment 310. Optionally, this Is achieved by use of valve 100. Optionally, an actuator 200 additionally regulates valve 100. Optionally, regulation is via a defined duty cycle. Valves 100 of the type pictured in Figs. 1A and 1B have an average gas release rate of 5 ml/s when applied to a 5 ml gas source containing 2.5 grams of CO₂ as described hereinabove. This means that an actuator 200 with a duty cycle of 1s/8 hrs (open to closed) would allow valve 100 to deliver an average of 15 ml of gas per day into expandable compartment 310 so that expansion of 1200 ml is achieved in six months. Adjustment of the duty cycle could be used to achieve very low fill rates (e.g. 1s/week for 0.71 ml/week) or higher fill rates (e.g. 5s/day for 75 ml/day). Alternatively or additionally, duty cycle may have two more than one phase (e.g. 1s/8hrs but with operation only on alternate days. The invention is very flexible in this regard and virtually any desired fill program may be implemented by adjusting the fill program and/or duty cycle. The duty cycle can be adjusted so that a higher or lower average daily expansion rate is achieved. An "on" period in the millisecond range seems feasible from an engineering standpoint considering fractional characteristics of valve 100 and actuator 200. High frequency actuation with a duty cycle including primarily the closed phase is within the scope of the invention, In an exemplary embodiment of the invention, a release of 1 to 10 ml of gas in a single valve actuation causes a minimal increase in pressure in compartment 210 which returns to base line over time. Optionally, the return to baseline results from tissue expansion. Although actuator 200 causes inflation to be incremental, the increments are optionally frequent and/or small so that they are not perceived by the subject in whom device 300 is implanted. In an exemplary embodiments of the invention open loop expansion reduces the need for clinic visits for inflation. Optionally, the open loop does not provide a linear expansion rate throughout the treatment period.

In an exemplary embodiment of the invention, expansion of comportment 310 creates an expansion pressure on the surrounding tissue of 10 to 200 mm of mercury, optionally, 20 to 150 mm of mercury, optionally, 30 to 100 mm of mercury, optionally, 50 to 85 mm of meroury. Optionally, this pressure may be maintained overtime and/or applied in discrete expansion events with intermittent pressure reductions resulting from tissue expansion. According to various embodiments of the invention, a desired expansion pressure may vary depending upon the tissue to be stretched and/or a condition of the tissue and/or the degree of expansion required and/or subject age and/or a desired treatment duration.

In an exemplary embodiments of the invention, expansion of the expandable compartment 310 is performed according to a program in which gas is periodically released so that no conscious cooperation of the subject is required. Programs might be defined, for example, in terms of time and/or number of actuation cycles and/or amount of gas permitted to flow through valve 100 and/or amount of incremental inflation of compartment 310.

In an exemplary embodiment of the invention, a program which opens valve 100 for a fixed number of times (e.g. 1-10) per day might be implemented.

Optionally, the program is designed so that the transfer of gas into compartment 910 occurs in a way that the subject in whom the device is implanted does not perceive the expansion as it occurs (e.g inflation periods concentrated at night when subject is sleeping). A program of this general type reduces the need for clinic visits for inflation. Optionally, the program is implemented using a microsprocessor and/or electronic circuitry and/or mechanical means.

In an exemplary embodiment of the invention, the program controls release of contents of multiple gas sources 210. Release may optionally be sequential with fixed or varying release intervals. Each of sources 210 may be emptied in response to a signal. Optionally, sources 210 have either similar or different amounts of gas stored therein. Optionally, delivery of gas to compartment 310 in this way may be according to a fill profile described by any desired function in an exemplary embodiment of the invention, the fill profile is designed to keep expansion in compartment 310 relatively constant and more gas/day is delivered later in the treatment program when compartment 310 is larger. Optionally, no valve 100 is required when multiple gas sources 210 are employed. An example configuration of an implantable device that contains multiple chambers, each individually addressable, for example by a separate wire and each containing a gas source or gas generating chemical reactants, would be the silicon chip based system developed by MicroChips (www.mchips.com). Optionally, a single use tear valve may be employed. Optionally, because gas is compliant, relatively large volumes may be added in a single fill event without causing subject discomfort. In an exemplary embodiment of the invention, a single fill event delivers a gas volume corresponding to 5 to 50, optionally 6 to 30, optionally 7.5 to 20, optionally about 10 to 15% of a volume of compartment 310 prior to the fill event. Optionally, the compliant nature of gas permits compartment 310 to undergo a slight increase in pressure instead of increasing in volume. This can prevent tissue damage because it permits the tissue to stretch slowly in response to a fill event, as opposed to liquid based or gel based expansion which transfers expansion force to the tissue immediately.

In general, pressure inside compartment 310 is controlled by Boyles' gas law that relates the initial volume of the expander, the final volume of the expander, the temperature of the gas (typically body temperature, i.e. 37C constant) and the incremental molar addition of gas delivered from source 210 to the compartment 310. The moles of gas delivered are a function of the initial pressure differential between source 210 to the compartment 310, the flow rate through the flow restrictor, and the time valve 100 is kept in the open position. The flow rate should be kept low enough to insure that if valve 100 fails overflow valve 323 can safely vent the gas. Optionally, a desired flow rate may vary with tissue type and site of implantation. In general, a desired flow rate may be less then the insuflation rates practiced in laparoscopic surgery. Venting through valve 323, if required, may optionally be into the atmosphere (optionally via a tube to the surface of the skin) and/or to the subcutaneous tissue. Venting to subcutaneous tissue is optionally at a rate which permits initial tissue expansion serious injury.

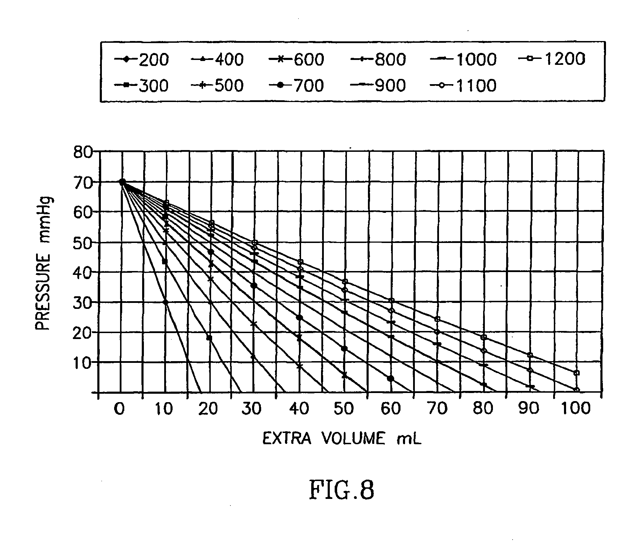

Fig. 8 is a graph illustrating the force of expansion pressure created as a function of an incremental additional volume of gas for compartments 310 with initial volumes in the range of 200-1200 ml (each initial volumes is plotted as a separate line on the axes). The graph presumes a desired target pressure after inflation of 70 mm of mercury above atmospheric pressure, but a similar graph could be prepared for any desired target pressure. Alternatively or additionally, the graph of Fig. 8 is for CO2, but a similar graph could be prepared for any desired gas. For any known internal pressure (e.g. 45 mm mercury) of compartment 310 on the Y axis, a line extending rightwards will intersect an initial volume line. A vertical line drawn to the X axis indicates a volume of CO2 required to achieve the target pressure of 70 mm of mercury.

For example, a compartment 310 with a 300 ml initial volume (filled squares) and an initial expansion pressure of 45 mmHg will require an incremental addition of 10 ml of gas to achieve the desired 70 mmHg expansion pressure. For a larger compartment 310 (e.g. 500 ml) the expansion pressure increase would require a larger volume of added gas (e.g. 30 ml). While any target expansion pressure may be theoretically achieved, an expansion pressure in the range of 5 to 150, optionally 10 to 120, optionally 30 to 80, optionally 40 to 70 optionally about 50 mm of mercury is typically desired for tissue expansion. The exact pressure desired for expansion may vary according to the subject and/or the tissue to be expanded and/or the condition of the tissue to be expanded. An expansion pressure that causes long term ischemia is generally to be avoided. In an exemplary embodiment of the invention, a subject assesses their own tissue ischemia using, for example, a threshold of discomfort as a guideline. In an exemplary embodiment of the invention, a clinician assesses ischemia, for example by assessing tissue texture and/or coloration and/or pressure response and/or texture, during a clinic visit and adjusts a treatment plan accordingly.

In an exemplary embodiment of the invention, device 300 supplies a controlled expansion pressure on a tissue through expansion of compartment 310. The desired expansion pressure may optionally be a pressure profile which may be optionally be fixed or dynamic. The desired pressure profile may vary according to factors set forth hereinabove. Optionally, one or more sensors 330 provide a measure of tissue ischemia and/or tissue tension. These sensor outputs may be used in implementation of a feedback loop and/or stored and/or transmitted to a remote data base. Analysis of stored data and/or comparison to a database optionally permits adjustment of the pressure profile. Optionally, a data processor (e.g. digital or analog) performs analysis and/or a profile adjustment within device 300. Examples of analog processors include, but are not limited to, ASIC devices and/or a power amplifier feedback circuit. In an exemplary embodiment of the invention, sensor 330 collects tension/pressure data periodically and/or continuously throughout the day to insure that prolonged periods of over pressure. This may be important in preventing ischemia, especially if device 300 is implanted between or beneath contractile muscles (e.g. pectoralis muscles for breast expansion device). Optionally, a doctor can input a desired pressure level, optionally in accord with a subject specific pressure profile, and device 300 will implement a feedback loop to maintain adjust compartment 310 to the desired pressure level. Optionally, adjustment is periodic (e.g. several times per day) or continuous. In an exemplary embodiment of the invention, this reduces tissue damage (e.g. scarring or stretch marks) during tissue expansion.

Alternatively or additionally, the subject in whom the device is implanted may control expansion of device 300 by means of actuator 200, for example by using an external control unit 350 (Fig. 3A) or by manipulation of a sub dermal switch which activates actuator 200. Subject mediated control may employ, for example, an open loop or program mode of operation as described hereinabove. In an exemplary embodiment of the invention, the subject employs a magnet in proximity to actuator 200 and valve 100 remains open as long as the magnet remains in position.

In an exemplary embodiment of the invention, a subject might press a button 360 (Fig. 3A) on external control unit 350 to trigger an inflation event (e.g. by issuing an operational command). Optionally, the inflation event would rely upon an open loop system in which actuator 200 receives an activation signal which opens the loop. The loop may optionally remain open as long the activation signal continues (e.g. as long as the button is pressed). In an exemplary embodiment of the invention, the subject prevents discomfort by ending the activation signal. Optionally, no duty cycle is applied and gas flows through valve 100 unrestricted, e.g. at a rate of approximately 5 ml/ second. Optionally, a single activation signal to actuator 200 opens the loop for a preset amount of time (e.g. 3 seconds), or a preset flow volume through valve 100 (e.g. 15 ml), or causes emptying/activation of a single gas source 210. In an exemplary embodiment of the invention, imposition of a finite limit on the response to the activation signal serves as a safety feature. Optionally, a feedback loop permits a single activation signal to cause gas transfer to compartment 310 until a physiologic response is received (e.g. skin stretch). Optionally, the feedback loop provides an additional level of safety.

Optionally, the activation signal might activate an inflation program of the general type described hereinabove. For example, if a subject presses button 360 one time before going to sleep the 2.4 hour inflation cycle divided into 14.4 minute incremental inflation periods might be activated. Optionally, a first incremental inflation period might begin after a delay of, for example 40 minutes, in order to give the subject time to fall asleep. Subject mediated control of the device may reduce the need for clinic visits for inflation. Alternatively or additionally, subject mediated control may increase subject satisfaction with the tissue expansion procedure and/or device 300.

In an exemplary embodiment of the invention, actuator 200 is responsive to at least one parameter of expandable compartment 310. Parametric measurement of the operational state of compartment 310 permits additional control of a degree of inflation of compartment 310. Responsiveness may be achieved by use of a parametric sensor 330. Sensor 330 may be attached to a wall of, or deployed within, expandable compartment 310. Relevant parameters for measurement include, but are not limited to, inflation pressure, tension in the surface of compartment 310 or volume of compartment 310.

Optionally, a volume of compartment 310 may be calculated by multiplying a flow rate through valve 100 by a time that valve 100 has been open, adding an initial volume of compartment 310 and subtracting any applicable leakage.

Fig. 3C illustrates a parametric sensor 330 to sense inflation pressure of compartment 310. Sensor 330 includes a pressure transducer coupled to a reference pressure source 331 and a pressure regulator 332 with a set point. These components control actuator 200 and valve 100 through a controller 385, optionally in response to a signal from external control unit 350.

Pressure sensor 330 regulator is formed by having a deformable reference chamber 331 with a fixed volume gas sealed within. Chamber 331 is exposed to the pressure inside the compartment 310 and will thus expand if the pressure inside compartment 310 is lower than the reference gas inside the reference chamber or contract if the opposite is true. The movement of the deformable portion of the reference chamber is connected to a valve (e.g., elastomeric seal 125) and thus modulates the flow out of source 210 into compartment 310. For example, as the pressure compartment 310 drops, reference chamber 331 expands relieves the pressure it exerts on elastomer seal 125 pressed against capillary 140 so that gas flows from source 210 to compartment 310. When enough gas has flowed and the pressure inside the compartment 310 rises sufficiently to compress deformable reference chamber 331 back to its original shape, it closes the valve. In this way homeostasis is established through a mechanical feedback loop. Optionally, the set point may be adjusted through a magnetic adjustment mechanism 333.

Alternatively or additionally, inflation pressure and/or tension in the surface of compartment 310 may be determined using commercially available devices. (e.g. Honeywell microstructure Pressure Sensor 26PC, Freeport IL, USA). One of ordinary skill in the art will be able to incorporate existing sensors into the context of device 300.

In an exemplary embodiment of the invention, actuator 200 may be responsive to at least one parameter of a body of the subject. Parametric measurement of subject response permits additional control of a degree of inflation of compartment 310. A measured subject body parameter might include, for example, a measure of blood perfusion of tissue covering device 300, a state of subject activity (e.g. as measured by an accelerometer, pulse monitor or respiratory monitor). Optionally, optical or other means of assessing the capillary blood flow in the skin over device 300 and increasing the expander's internal pressure up to the point of assuring safe blood circulation in the expanded skin are employed. Parametric sensor 330 for measuring blood perfusion may be external (patient holds it or it is taped over the skin) incorporated with device 300 or controller 350. For example, a colorimetric sensitive photo detection means may be employed to assess the level of perfusion in the surrounding tissue. Optionally, periodic measurements are employed as a power conservation measure.

Alternatively or additionally, sensor 330 may rely upon an ultrasonic transducer that measures speed changes in an ultrasonic wave caused by tissue. Optionally, speed changes are related to tissue tension.

Optionally, device 300 and/or control unit 350 include a digital processor (e.g. microprocessor) and/or an analog mechanism and/or a mechanical mechanism to monitor parametric data and/or control actuator 200. In an exemplary embodiment of the invention, a parametric feedback loop is implemented without a physical percutaneous link, for example through controller 350.

In an exemplary embodiment of the invention, a feedback loop whether relating to an operational status of compartment 310 or a patient response, imposes defined limits on expansion of expandable compartment 310 and/or increases an operational safety of device 300. Optionally, subject discomfort is comfort is decreased and/or treatment efficacy is increased and/or the time to achieve a desired degree of tissue expansion is reduced.

Optionally, it may be desirable to allow and/or require subjects to provide a signal, for example by means of control unit 350. In an exemplary embodiment of the invention, the patient receives feedback concerning an inflation status of compartment 310, for example as visual output on controller 350. Optionally, this feedback increases patient compliance and/or satisfaction. Optionally, conscious control of expansion is facilitated by this feedback. Optionally, the subject initiates a manual feedback loop based upon this feedback.

In an exemplary embodiment of the invention, signals from a remote location may be routed through control unit 350 using a relay device 390. The relay device may include, for example, a communication antenna 390 and/or a data port 390. Relay device 390 is optionally connectable to a computer and/or a telephone network and/or a specific telephone line. Optionally, port 390 facilitates data transfer to a remote computer 650. Optionally, data transfer is through a WAN such as the Internet Optionally, the Bluetooth communication protocol facilitates data transfer between controller 350 and/or device 300 and remote computer 650.

In an exemplary embodiment of the invention, data from parametric sensor 330 of device 300 is routed through a wire 321 to an antenna (e.g. an RF coil) 320 mounted on a wall of compartment 310. Optionally, antenna 320 is mounted inside compartment 310 as shown in Fig. 3D. Optionally, this is accomplished by sandwiching between 2 layers of material as pictured. Alternatively or additionally, connection 321 between antenna 320 and actuator 200 follows the contour of compartment 310. Optionally, anchoring studs 311 help insure that antenna 320 remains close to the skin surface and/or in a known location. Optionally, source 210 and/or actuator 200 are anchored to base 301 with retention straps 221, clearly visible in Fig. 3D.

A companion antenna 322 on controller 350 may optionally be capable of receiving this signal. In an exemplary embodiment of the invention, signals between the control unit 350 and device 300 are delivered without a physical percutaneous link. These signals may be from the device to the control unit and/or from the control unit to the device. Optionally, the signal includes power and/or data. In order to conserve power and/or to prevent accidental signaling, antennae 320 and 322 may be configured to work only over very short distances (e.g. 5 to 25 mm). Optionally, antennae 320 and 322 are circular and function as coils with near field coupling. In an exemplary embodiment of the invention, the control unit is small and portable and may be operated by either a doctor or by the subject in whom the device is implanted. Alternatively or additionally, antenna 322 may include induction coils which may be used to power operative components of device 300, such as actuator 200.

In a first experiment, a controller with an antenna 322 was used to broadcast a signal to a matched antenna 320 over a distance of 15 mm with an input power of 230 mW. Results are summarized in Table 1.

**Table 1: DC power developed from a 230 mW RF power input delivered from a distance of 15 mm.**

| Load resistance | DC voltage developed (V) | Power to load (mW) |
|---|---|---|
| 600 | 3.5 | 20 |
| 300 | 2.4 | 19 |
| 150 | 1.4 | 13 |

In an additional experiment, a controller with an antenna 322 was used to broadcast a signal to a matched antenna 320 over a distance of 6.25 mm with an input power of 320 mW. Results are summarized in Table 2.

**Table 2: DC power developed from a 320 mW RF power input delivered from a distance of 6.25 mm.**

| Load resistance | DC voltage developed (V) | Power to load (mW) |
|---|---|---|
| 600 | 3.5 | 20 |
| 300 | 2.7 | 24 |
| 150 | 2.0 | 26 |

These experiments suggest that external controller 350 provides a safe and reliable means of controlling transfer of gas from the gas source 210 to expandable compartment 310 by separating the power source from actuator 200. This assures that actuator 200 operates only when controller 350 is in close proximity to device 300, thereby preventing accidental inflation of compartment 310 of device 300. Data presented in tables 1 and 2 are exemplary only and actual transfer of power between controller 350 and device 300 is expected to be more efficient and/or more stringently controlled with respect to distance.

As an additional or alternate safety precaution, antenna 322 in controller 350 may be keyed to one or more devices 300 in order to control who controls which device(s). Keying may be, for example, via frequency matching, cryptography or other known recognition methods.

In an exemplary embodiment of the invention, a doctor uses a single wireless controller 350 to operate devices 300 implanted in several subjects. This simplifies matters for the doctor and does not preclude a subject from using a control unit 350 keyed only to their own device 300.

In an exemplary embodiment of the invention, wireless control unit 350 includes a signal receiver 322 which receives information from a parametric sensor 330 on performance of device and/or a subject parameter. Receiver 322 may optionally receive input concerning parametric measurement of expandable compartment 310 and/or subject the subject. microprocessor 385 in device 300 translates parametric measures into required inflation volume or time to operate actuator 200. Because size limitations on external control unit 350 are less stringent, microprocessor 385 may handle more of the workload Optionally, control unit 350 relays gathered information on device performance to a remote location (e.g. server 650; Fig. 5) for medical supervision and/or statistical analysis via communication channel 630. Optionally, the same communication channel relays data to device 300, optionally through controller 350. This may facilitate, for the first time, a database which correlates subject response to objective operational data on an implanted tissue expansion device 300. Subject response may be objective (e.g. parametric data) and/or subjective (e.g. subject rating of discomfort on a scale of 1-10).

In an exemplary embodiment of the invention, a subject with an implanted tissue expander periodically uploads data from their device 300 to a remote server 650. This may be accomplised, for example by transferring parametric data from parametric sensors 930 to data storage device 380 via matched antennae 320 and 322. Data storage device 380 may optionally be a small device with limited capacity, such as a flash memory card, a chip or a SIM card. Optionally, data may be temporarily stored within device 300 and/or controller 350. In an exemplary embodiment of the invention, data pertains to patient compliance and may indicate, for example a number of times that a patient initiates a trigger event for fill of compartment 310. Alternatively or additional, patient compliance may be measured in terms of a total expansion rate of compartment 310.

Power for this transfer may be provided by one or more power sources 370, optionally by a power source 370 in control device 350. Upload may occur, for example, via data transfer device 390 via, for example a cellular telephone connection or an Internet connection. Optionally, control device 350 is configured as a cellular telephone. Optionally, a subject's existing cellular telephone is transformed into a control device 350 by appropriate software installation. Optionally, control device 350 issues reminders to a subject via a reminder mechanism. Optionally, thE reminders encourage compliance with a tretment plan. Reminders may be, for example, visual, tactile or audible (e.g. flashing light, information on display screen, vibration or distinctive tone played through a speaker). Alternatively or additionally, control device 350 includes a display for data from one or more sensors 330.

The remote server may optionally issue an instruction to device 300 based upon analysis of the uploaded data. Optionally, a doctor reviews the uploaded data and transmits the instruction via server 650 to controller 350 for relay to device 300. Optionally, this permits modification of a treatment plan without a clinic visit.

### Informatics applications:

Because sensors 330 provide a convenient means for data acquisition and/or storage and/or transfer, an exemplary embodiment of the invention relates to a computer designed and configured to respond to queries concerning performance of implantable tissue expansion devices. The computer 650 includes a memory containing data pertaining to:(i) design feature data pertaining to at least one implantable tissue expansion device; (ii) operational data pertaining to at least one implantable tissue expansion device employed for treatment in an individual subject; and (iii) subject data pertaining to a response of said individual subject in said implantable tissue expansion device has been implanted. Optionally, compliance data pertaining to a treatment program compliance of individual subjects in whom a device 300 has been implanted is also stored. In an exemplary embodiment of the invention, data is concurrently collected on a large number of implanted devices. Optionally, the devices implement similar and/or different treatment plans. Optionally, the devices are of similar and/or different design. The computer 650 contains circuitry configured to receive a query and formulate a response based upon data stored in the memory. Computer 650 is optionally a server, optionally accessible across a WAN.

In an exemplary case, computer 650 facilitates a method of determining a desirable design characteristic of an implantable tissue expansion device, the method comprising analyzing data from a database and identifying at least one design feature which correlates to a favorable subject response.

In an exemplary case, computer 650 facilitates a method of determining a desirable tissue expansion program for use in conjunction with implantable tissue expansion devices of a given design, the method comprising analyzing data from a database and identifying at least one operational parameter which correlates to a favorable subject response.

In an exemplary case, data acquisition is automated by a computerized system designed and configured to construct a database of implantable tissue expansion device data. The system includes:(a) a design feature data acquisition module to acquire and store design feature data pertaining to a plurality of implantable tissue expansion devices; (b) the implantable tissue expansion devices designed and configured to acquire and store data on at least one device performance characteristic; (c) a plurality of subject data parametric sensors designed and configured to acquire and store data on at least one subject response parameter; and (d) a data relay connectable to each device and/or parametric sensors for purposes of transferring data a memory in computer 650.

These informatics examples are expected to spur design development of a new generation of tissue expansion devices and/or increase efficacy of treatment programs using existing devices. The computerized systems described herein permit systematic analysis of treatment response to tissue expansion in a way not previously possible.

### Exemplary Safety features

Because device 300 is implanted within a living subject, safety of the subject is of high importance.

Configurations of device 300 which do not include any percutaneous link or port are generally safer than any configurations which include a percutaneous link or port from the standpoint of infection.

Devices 300 which rely upon a gas source 210 are inherently self limiting in terms of total inflation. The maximum total inflation is a function of the amount of gas present in source 210 when device 300 is implanted.

Alternatively or additionally, the compliant nature of gas permits compartment 310 to undergo a slight increase in pressure instead of increasing in volume. This can prevent tissue damage because it permits the tissue to stretch slowly in response to a fill event, as opposed to liquid based or gel based expansion which transfers expansion force to the tissue immediately. As a result, a fill volume which causes only a small degree of immediate compartment expansion with a subsequent further expansion in response to expansion of overlying tissue can be calculated and implemented based upon the specific gas employed, the current compartment volume and/or pressure and the characteristics of the surrounding tissue.

Additional safety may be achieved by actuator and/or valve configuration. Valves 100 with lower flow rates may be safer than those with higher flow rates. Valves with low flow rates afford a subject more time to seek intervention or aid in case a valve fails in an open position. Similarly, actuators which naturally tend to assume a closed position may be safer than actuators which naturally tend towards an open position. These actuators insure that if power becomes unavailable, a flow of gas through valve 100 will be stopped. While this may interfere with planned tissue expansion, it reduces a potential danger to the subject In an exemplary embodiment of the invention, valve/actuator combinations which rely on infrequent opening of the valve for a short period of time impart a high reliability to device 300. Since it is possible to calculate the total number of valve openings required to release all of the gas in a source 210 into compartment 310, a valve/actuator combination which has been tested for 10, optionally 100, optionally 1000 or more actuation cycles than actually required may be employed.

As detailed hereinabove, parametric sensors may provide feedback loops. Optionally, feedback loops are under control of a microprocessor. Alternatively or additionally, a mechanical or electric feedback loop may be implemented by deploying a pressure sensitive switch between an elastic balloon and an inelastic shell. These loops may aid in preventing unwanted over inflation of compartment 310.

Optionally, compartment 310 leaks at a known rate. This means that if inflation is carried out to the point of discomfort, gradual relief will occur without any active intervention. Alternatively or additionally, a pressure sensitive valve releases excess pressure from compartment 310. Optionally, release of excess gas is into the body and/or transdermal.

Alternatively or additionally, use of an external power source with a short operational distance may prevent unwanted filling or inflation of compartment 310. Exemplary embodiments of power sources with an effective operational range of only a few millimeters are described hereinabove. These power sources prevent filling of compartment 310 unless the power source is brought into close proximity to device 300.

Alternatively or additionally, a release valve 323 (Fig. 3A) is provided to prevent excessive expansion pressure in compartment 310. Optionally, gas is released through valve 323. Optionally, gas is released into the body. Optionally, gas is released through a percutaneous release valve. In an exemplary embodiment of the invention release port 323 is an over pressure relies valve 323. Over pressure condition in side the expander optionally cause release through valve 323 by mechanical means and/or through control implemented via a microprocessor.

Optionally, coating is applied inside compartment 310. Optionally, the coating includes Surfacine®. Optionally, a coated substance such as non-woven polymeric 'wool' may be placed within the volume of the expander. Optionally, this may increase the ratio of coated surface to volume. Optionally, this improves antibacterial efficacy. Alternatively, an antimicrobial substance such as broad spectrum antibiotic or antimicrobial can be placed in compartment 310.

Optionally, external surfaces of device 300 are treated with an antibacterial substance. Optionally, treatment is in the form of a non-eluting coating, such as, for example Surfacine or a Surfacine like compound. Alternatively or additionally, the coating includes an eluting material, such as, for example, an antimicrobial compound such as silver or an antibiotic. Optionally, a hybrid eluting/non eluting coating is employed. In an exemplary embodiment of the invention, application of an antimicrobial coating prevents or rotards formation of a biofilm. Alternatively or additionally, an antimicrobial coating prevents a coated portion of device 300 from becoming a source of infection.

In an exemplary embodiment of the invention, no peroutaneous fill port is employed. Optionally, this reduces the risk of clinical and/or sub-clinical infection from filling via the percutaneous port. A subclinical contamination, if it were to occur, may promote formation of a scar capsule around the implant. A scar capsule might inhibit successful subsequent expansion of the implant. Alternatively or additionally, in breast reconstruction embodiments in which tissue expansion device 300 is to be replaced by a permanent cosmetic implant, contamination might be passed to the permanent implant. This transfer has the potential to cause delayed capsular problems and/or overt inflection.

### Aesthetic considerations

Optionally, it may be desirable for device 300 to impart a natural body contour, for example in breast reconstruction. Optionally, an initial volume may be imparted to compartment 310 to provide a shape suggestive of a contralateral organ. Optionally, the initial volume may be supplied from gas source 210 shortly after implantation. In an exemplary embodiment of the invention, disruption of a body contour is reduced by reducing a size of at least a portion of device 300.

Alternatively or additionally, compartment 310 may be partially filled prior to implantation. Partial fill may be accomplished by introducing gas and/or a liquid and/or a geI into compartment 310. Optionally, the partial fill is sequestered in a sub compartment within compartment 310 or in a separate compartment within device 300. In an exemplary embodiment of the invention, a percutaneous fill port is provided to facilitate the partial fill which may optionally be an initial volume and/or a supplementary volume and/or a desired gravitational characteristic.

Optionally, the percutaneous fill port is used to remove filling of compartment 310(e.g. gas). In an exemplary embodiment of the invention, compartment 310 is emptied via the percutaneous fill port and refilled with a substance (e.g. silicone gel or saline) that transforms device 30D into a long-term implant. In an exemplary embodiment of the invention, conversion of device 300 into a long term implant eliminates an additional surgical procedure, thereby reducing scarring of the subject and/or obviating a need for additional tissue resection.

As explained hereinabove compartment 310 may be formed from a deformable inolastic material which is premolded to a desired shape. This may bo accomplished, for example, by welding or vacuum molding two sheets of material together. Alternatively or additionally, pleats or folds may be wed to impart a desired shape. Desired shapes optionally include partial spheres (e.g. hemisphere), offset partial sphere or breast (tear) shaped.

In an exemplary embodiment of the invention, a plurality of compartments 310 are provided in a single device 300. Optionally, these compartments 310 are filled concurrently or sequentially. Optionally, each compartment 310 has its own gas source 210 or compartments 310 share a common gas source 200 but are individually valved and/or actuated Such an arrangement optionally provides control over the direction of expansion in all three dimensions and over time. In an exemplary embodiment of the invention, this permits tissue expansion to be directed first to one side and then the other. This may be desirable if anchoring studs, as describe hereinbelow, are employed.

In an exemplary embodiment of the invention, struts that break at a defined stress and/or different resistance to expansion in different parts of the device may be employed to impart a desired conformation to compartment 310 during expansion.

In implant breast reconstruction it is desirable to achieve medial expansion while not over-releasing the medial aspects of the pectoralis muscle. Over-release of the muscle causes a post-operative condition known as "window-shading" in which released ends of the muscle contract towards the shoulder and pull the overlying breast tissue accordingly. Whenever the subject adducts the arm there is a profound distortion of the breast. At the same time, inadequate release of the medial aspects of the muscle causes the tissue expander to 'sit' in a lateral position during placement and/or to be pushed laterally in the post-operative period by muscle contraction. Thus, although "window shading" might be avoided by preserving the medial origins of the pectoral muscle, the resulting breast expansion often occurs in an inappropriate position. Either of these results is aesthetically unacceptable and means to avoid these problems are desired.

In an exemplary embodiment of the invention, device 300 is anchored so that its position remains stable even if release of the medial portion of the pectoralis major origin is partially preserved. Previously available anchoring strategies relied upon a textured surface to prevent shifting of an implanted body relative to surrounding tissue. Because protrusions on a roughened surface were typically micro-protrusions, a large number of protrusions were typically applied. This contributed to string diffuse attachment which made removal of the implanted body difficult.

In an exemplary embodiment of the invention, a studded surface is employed for anchoring so that protruding studs penetrate the overlying pectoralis muscle in order to prevent movement of device 300 with respect to the muscle. Optionally, studs are installed on an anterior surface. Optionally, 1-500, optionally, 2-350, optionally 3 to 75, optionally 4 to 50, optionally 5 to 25, optionally 6-10 studs of 2-3 mm in height are sufficient for anchoring. In an exemplary embodiment of the invention, the small number of studs provides a desired degree of anchoring but does not contribute to difficulty in removing device 300. Optionally, the studs are resorbable. In an exemplary embodiment of the invention, once a capsule has formed to stabilize the position of device 300, the studs are resorbed.

Some portions of the invention rely upon execution of various commands and analysis and translation of various data inputs. Any of these commands, analyses or translations may be accomplished by software, hardware or firmware according to various embodiments of the invention. In an exemplary embodiment of the invention, machine readable media contain instructions for formulation of a treatment recommendation including an inflation recommendation with a volume component in response to one or more measures of subject response and or performance of device 300 is provided. In an exemplary embodiment of the invention, microprocessor 385 executes instructions for inflation of compartment 310 by translating a received inflation volume input into a set of instructions for actuator 200.

Device 300 may be employed, for example in breast reconstruction, facial reconstruction, expansion of visceral tissues, nerves, smooth muscle, striated muscle, cardiac muscle, blood vessels or connective tissue. In an exemplary embodiment of the invention, device 300 may be employed in reconstructive surgery after accidents and/or amputations. Alternatively or additionally, device 300 may be installed as an intramedullary device for bone lengthening. For example a device 300 placed in the forearm under blood vessels, nerves, investing fascia and skin expands gradually so that all of these structures increase their dimensions. When expansion is complete, it is possible to transplant a composite tissue as a flap including each of the individual tissues. The blood vessels may be anastomosed to blood vessels at the recipient site in order to supply the transplanted flap with a blood supply. The nerve may be coapted to nerves in the recipient site to offer sensation and/or motor control to the flap; and the investing tissue and skin offer a stable coverage to the recipient site.

Device 300 may optionally be employed to remold tissue. Remolding includes, but is not limited to, tightening bone and/or ligament and/or creating a void inside the body for implantation of a device. Optionally, device 300 offers the possibility of creating a cavity well below the skin surface so that a subsequent device might be implanted without altering body contours. In an exemplary embodiment of the invention, the cavity is used for implantation of a medical device such as a pump for sustained release of medication.

Various embodiments of the invention rely upon execution of various commands and analysis and translation of various data inputs. Any of these commands, analyses or translations may be accomplished by software, hardware or firmware according to various embodiments of the invention. In an exemplary embodiment of the invention, machine readable media contain instructions for a tissue expansion treatment plan and/or data pertaining to device performance and/or subject response and/or subject compliance is provided.

In the description and claims of the present application, each of the verbs "comprise", "include" and "have" as well as any conjugates thereof, are used to indicate that the object or objects of the verb are not necessarily a complete listing of members, components, elements or parts of the subject or subjects of the verb.

In the description and claims of the present application, some components and/or parts are depicted as separate elements for clarity although their functions might be combined in a single physical entity in actual practice.

The present invention has been described using detailed descriptions of embodiments thereof that are provided by way of example and are not intended to necessarily limit the scope of the invention. In particular, numerical values may be higher or lower than ranges of numbers set forth above and still be within the scope of the invention. The described embodiments comprise different features, not all of which are required in all embodiments of the invention. Some embodiments of the invention utilize only some of the features or possible combinations of the features. Variations of embodiments of the present invention that are described and embodiments of the present invention comprising different combinations of features noted in the described embodiments can be combined in all possible combinations including, but not limited to use of features described in the context of one embodiment in the context of any other embodiment The scope of the invention is limited only by the following claims.

## Claims

1. A tissue expansion device, the device comprising:
(a) an expandable compartment (310) adapted for implanting in a body of a subject; and
(b) a compressed gas source (210) operably communicating with said expandable compartment (310) for inflation thereof by transfer of a gas thereto,
**characterized in that** said compressed gas source (210) is positioned completely within said expandable compartment (310).

2. A device according to claim 1, **characterized in that** said expandable compartment (310) is at least partially constructed from an inelastic material.

3. A device according to any of the preceding claims, **characterized in** additionally comprising: a regulator (100,200) configured to enable multiple incremental transfers of gas from said gas source (210) into said expandable compartment (310) to controllably inflate the expandable compartment (310).

4. A device according to claim 3, **characterized in that** said regulator (100,200) includes a flow restriction pathway.

5. A device according to any of claims 3 or 4, **characterized in that** said regulator (100,200) includes a valve (100) to restrict a flow of gas from said gas source (210) to said expandable compartment (310).

6. A device according to claim 3, **characterized in that** said regulator (100,200) is responsive to an input signal.

7. A device according to claim 6, **characterized in that** said input signal includes an operational command originating from an external controller (350).

8. A device according to 6, **characterized in that** said input signal includes an output signal from at least one sensor (330).

9. A device according to claim 8, **characterized in that** said sensor is configured to measure at least one parameter of the expandable compartment (310), and wherein said at least one parameter of the expandable compartment (310) includes a measure of a gas pressure within said compartment (310).

10. A device according to claim 1, **characterized in** additionally comprising: a power source (370) disposed external to the subject and configured to provide power to the tissue expansion device in a wireless manner.

11. A device according to any of the preceding claims, **characterized in** additionally comprising: an outer shell surrounding said expandable compartment (310).

12. A device according to claim 2 , **characterized in that** said inelastic material has an expanded shape contoured to a human breast shape.

13. A device according to any of the preceding claims, **characterized in** additionally comprising: a release valve (323) configured to release gas from said expandable compartment (310).

14. A device according to any of the preceding claims, **characterized in that** the device is configured for expansion of surrounding tissue to a desired expansion state, and wherein the inflation of said expandable compartment (310) results in controllable expansion of surrounding tissue to said desired expansion state.

15. A device according to any of the preceding claims, designed and configured to discharge gas from said gas source (210) into said expandable compartment (310) during a period of at least 7 days.

16. The device of claim 1, **characterized in that** the gas source (210) operably communicates with said expandable compartment (310) for controllable inflation thereof.

17. The device of claim 14, **characterized in that** the surrounding tissue is breast tissue and whereas said desired expansion state is that suitable for subsequent implantation of a breast implant.

18. The device of claim 3, **characterized in that** the regulator (100,200) is responsive to a wireless signal from a device (350) disposed external to the subject.

19. The device of claim 3, **characterized in that** the regulator (100,200) comprises a controllable actuator (200).

20. The device of claim 1, **characterized in** further comprising a narrow orifice (141) communicating the gas source (210) with the expandable compartment (310).

21. The tissue expansion device of claim 1, **characterized in that** the compressed gas source (210) comprises CO₂.

22. The device of claim 1, **characterized in that** the expandable chamber comprises a posterior backing, and wherein the compressed gas source (210) is secured to the posterior backing (301).

23. The device of claim 1, **characterized in** further comprising a communication component (320) secured to an anterior portion of the expandable chamber, the communication component adapted to wirelessly communicate with an external device (350).

## Patentansprüche

1. Gewebeexpansionsvorrichtung, wobei die Vorrichtung Folgendes umfasst:
(a) einen expandierbaren Raum (310), der ausgelegt ist, um in einen Körper eines Subjekts implantiert zu werden; und
(b) eine Druckgasquelle (210), die im Betrieb mit dem expandierbaren Raum (310) verbunden ist, um denselben durch die Übertragung eines Gases in denselben aufzublasen,
**dadurch gekennzeichnet, dass** die Druckgasquelle (210) vollständig innerhalb des expandierbaren Raum (310) positioniert ist.

2. Vorrichtung nach Anspruch 1, **dadurch gekennzeichnet, dass** der expandierbare Raum (310) mindestens teilweise aus einem nicht elastischen Material gebaut ist.

3. Vorrichtung nach einem der vorhergehenden Ansprüche, **dadurch gekennzeichnet, dass** sie zusätzlich Folgendes umfasst: einen Regler (100, 200), der konfiguriert ist, um zahlreiche inkrementelle Übertragungen von Gas aus der Gasquelle (210) in den expandierbaren Raum (310) zu ermöglichen, um den expandierbaren Raum (310) steuerbar aufzublasen.

4. Vorrichtung nach Anspruch 3, **dadurch gekennzeichnet, dass** der Regler (100, 200) einen Flussbeschränkungsweg umfasst.

5. Vorrichtung nach einem der Ansprüche 3 oder 4, **dadurch gekennzeichnet, dass** der Regler (100, 200) ein Ventil (100) umfasst, um einen Gasfluss aus der Gasquelle (210) in den expandierbaren Raum (310) zu beschränken.

6. Vorrichtung nach Anspruch 3, **dadurch gekennzeichnet, dass** der Regler (100, 200) auf ein Eingabesignal reagiert.

7. Vorrichtung nach Anspruch 6, **dadurch gekennzeichnet, dass** das Eingabesignal einen Betriebsbefehl umfasst, der aus einer externen Steuereinheit (350) stammt.

8. Vorrichtung nach Anspruch 6, **dadurch gekennzeichnet, dass** das Eingabesignal ein Ausgabesignal von mindestens einem Sensor (330) umfasst.

9. Vorrichtung nach Anspruch 8, **dadurch gekennzeichnet, dass** der Sensor konfiguriert ist, um mindestens einen Parameter des expandierbaren Raums (310) zu messen, und wobei der mindestens eine Parameter des expandierbaren Raums (310) eine Messung eines Geasdrucks in dem Raum (310) umfasst.

10. Vorrichtung nach Anspruch 1, **dadurch gekennzeichnet, dass** sie zusätzlich Folgendes umfasst: eine Stromquelle (370), die ausserhalb des Subjekts angebracht und konfiguriert ist, um auf drahtlose Weise der Gewebeexpansionsvorrichtung Leistung bereitzustellen.

11. Vorrichtung nach einem der vorhergehenden Ansprüche, **dadurch gekennzeichnet, dass** sie Folgendes umfasst: eine äussere Schale, die den expandierbaren Raum (310) umgibt.

12. Vorrichtung nach Anspruch 2, **dadurch gekennzeichnet, dass** das nicht elastische Material eine expandierte Form aufweist, die an die Form einer menschlichen Brust angepasst ist.

13. Vorrichtung nach einem der vorhergehenden Ansprüche, **dadurch gekennzeichnet, dass** sie Folgendes umfasst: ein Ablassventil (323), das konfiguriert ist, um Gas aus dem expandierbaren Raum (310) freizugeben.

14. Vorrichtung nach einem der vorhergehenden Ansprüche, **dadurch gekennzeichnet, dass** die Vorrichtung konfiguriert ist, um umgebendes Gewebe zu einem gewünschten Expansionszustand zu expandieren, und wobei das Aufblasen des expandierbaren Raums (310) zu einer steuerbaren Expansion des umgebenden Gewebes zu dem gewünschten Expansionszustand führt.

15. Vorrichtung nach einem der vorhergehenden Ansprüche, entworfen und konfiguriert, um Gas aus der Gasquelle (210) in den expandierbaren Raum (310) während eines Zeitraums von mindestens 7 Tagen freizugeben.

16. Verfahren nach Anspruch 1, **dadurch gekennzeichnet, dass** die Gasquelle (210) im Betrieb mit dem expandierbaren Raum (310) zum steuerbaren Aufblasen davon verbunden ist.

17. Verfahren nach Anspruch 14, **dadurch gekennzeichnet, dass** das umgebende Gewebe Brustgewebe ist, und wobei der gewünschte Expansionszustand für die folgende Implantation eines Brustimplantats geeignet ist.

18. Vorrichtung nach Anspruch 3, **dadurch gekennzeichnet, dass** der Regler (100, 200) auf ein drahtloses Signal von einer Vorrichtung (350) reagiert, die ausserhalb des Subjekts angeordnet ist.

19. Vorrichtung nach Anspruch 3, **dadurch gekennzeichnet, dass** der Regler (100, 200) eine steuerbare Betätigungsvorrichtung (200) umfasst.

20. Vorrichtung nach Anspruch 1, **dadurch gekennzeichnet, dass** sie weiter eine enge Öffnung (141) umfasst, die die Gasquelle (210) mit dem expandierbaren Raum (310) verbindet.

21. Gewebeexpansionsvorrichtung nach Anspruch 1, **dadurch gekennzeichnet, dass** die Druckgasquelle (210), CO₂ umfasst.

22. Vorrichtung nach Anspruch 1, **dadurch gekennzeichnet, dass** die expandierbare Kammer eine hintere Verstärkung umfasst, und wobei die Druckgasquelle (210) an die hintere Verstärkung (301) befestigt ist.

23. Vorrichtung nach Anspruch 1, **dadurch gekennzeichnet, dass** sie weiter eine Kommunikationskomponente (320) umfasst, die an einen vorderen. Abschnitt der expandierbaren Kammer befestigt ist, wobei die Kommunikationskomponente ausgelegt ist, um drahtlos mit einer äusseren Vorrichtung (350) zu kommunizieren.

## Revendications

1. Dispositif d'expansion tissulaire, le dispositif comprenant :
a) un compartiment expansible (310) apte à être implanté dans le corps d'un sujet ; et
b) une source de gaz comprimé (210) qui communique fonctionnellement avec ledit compartiment expansible (310) pour gonfler celui-ci en lui transférant un gaz,
**caractérisé en ce que** ladite source de gaz comprimé (210) est placée complètement à l'intérieur dudit compartiment expansible (310).

2. Dispositif selon la revendication 1, **caractérisé en ce que** ledit compartiment expansible (310) est constitué au moins en partie d'une matière inélastique.

3. Dispositif selon l'une quelconque des revendications précédentes, **caractérisé en ce qu'**il comprend en outre : un régulateur (100, 200) configuré pour permettre de multiples transferts progressifs de gaz de ladite source de gaz (210) dans ledit compartiment expansible (310) pour gonfler de manière commandée le compartiment expansible (310).

4. Dispositif selon la revendication 3, **caractérisé en ce que** ledit régulateur (100, 200) comprend une voie de limitation de l'écoulement.

5. Dispositif selon l'une quelconque des revendications 3 ou 4, **caractérisé en ce que** ledit régulateur (100, 200) comprend une soupape (100) destinée à limiter l'écoulement de gaz de ladite source de gaz (210) vers ledit compartiment expansible (310).

6. Dispositif selon la revendication 3, **caractérisé en ce que** ledit régulateur (100, 200) réagit à un signal d'entrée.

7. Dispositif selon la revendication 6, **caractérisé en ce que** ledit signal d'entrée comprend une commande de fonctionnement émanant d'un dispositif de commande externe (350).

8. Dispositif selon la revendication 6, **caractérisé en ce que** ledit signal d'entrée comprend un signal de sortie provenant d'au moins un capteur (330).

9. Dispositif selon la revendication 8, **caractérisé en ce que** ledit capteur est configuré pour mesurer au moins un paramètre du compartiment expansible (310), et dans lequel ledit au moins un paramètre du compartiment expansible (310) comprend une mesure d'une pression de gaz à l'intérieur dudit compartiment expansible (310).

10. Dispositif selon la revendication 1, **caractérisé en ce qu'**il comprend en outre : une source d'alimentation (370) placée à l'extérieur du sujet et configurée pour alimenter sans fil le dispositif d'expansion tissulaire.

11. Dispositif selon l'une quelconque des revendications précédentes, **caractérisé en ce qu'**il comprend en outre : une enveloppe externe entourant ledit compartiment expansible (310).

12. Dispositif selon la revendication 2, **caractérisé en ce que** ladite matière inélastique présente une forme expansée qui épouse la forme d'un sein humain.

13. Dispositif selon l'une quelconque des revendications précédentes, **caractérisé en ce qu'**il comprend en outre : une soupape de décharge (323) configurée pour libérer un gaz dudit compartiment expansible (310).

14. Dispositif selon l'une quelconque des revendications précédentes, **caractérisé en ce que** le dispositif est configuré pour assurer l'expansion du tissu environnant jusqu'à un état d'expansion souhaité, et dans lequel le gonflage dudit compartiment expansible (310) entraîne l'expansion commandée du tissu environnant jusqu'audit état d'expansion souhaité.

15. Dispositif selon l'une quelconque des revendications précédentes, conçu et configuré pour décharger un gaz de ladite source de gaz (210) dans ledit compartiment expansible (310) pendant une durée d'au moins 7 jours.

16. Dispositif selon la revendication 1, **caractérisé en ce que** la source de gaz (210) communique fonctionnellement avec ledit compartiment expansible (310) pour assurer le gonflage commandé de celui-ci.

17. Dispositif selon la revendication 14, **caractérisé en ce que** le tissu environnant est un tissu mammaire et dans lequel ledit état d'expansion souhaité est celui qui convient à l'implantation consécutive d'un implant mammaire.

18. Dispositif selon la revendication 3, **caractérisé en ce que** le régulateur (100, 200) réagit à un signal sans fil émanant d'un dispositif (350) placé à l'extérieur du sujet.

19. Dispositif selon la revendication 3, **caractérisé en ce que** le régulateur (100, 200) comprend un actionneur commandé (200).

20. Dispositif selon la revendication 1, **caractérisé en ce qu'**il comprend en outre un orifice étroit (141) qui met en communication la source de gaz (210) avec le compartiment expansible (310).

21. Dispositif selon la revendication 1, **caractérisé en ce que** la source de gaz comprimé (210) comprend du CO₂.

22. Dispositif selon la revendication 1, **caractérisé en ce que** la chambre expansible comprend un renfort postérieur, et dans lequel la source de gaz comprimé (210) est fixée au renfort postérieur (301).

23. Dispositif selon la revendication 1, **caractérisé en ce qu'**il comprend en outre un élément de communication (320) fixé à une partie antérieure de la chambre expansible, l'élément de communication étant apte à communiquer sans fil avec un dispositif externe (350).
